# EUROPÄISCHE PATENTANMELDUNG

(11) **EP 3 851 116 A1**
(43) Veröffentlichungstag der Anmeldung: **21.07.2021**
(21) Anmeldenummer: 20152663.9
(22) Anmeldetag: 20.01.2020
(51) Int. Cl.: A61K 36/66, A61K 31/17, A61K 31/35, A61K 31/351, A61K 31/4741, A61K 31/505, A61K 31/506, A61K 31/517, A61P 33/00, A23K 10/00

(54) **SUBSTANZKOMBINATION MIT ISOCHINOLINALKALOIDEN UND KOKZIDIOSTATIKUM ODER KOKZIDIOSEIMPFMITTEL**

(71) Anmelder: Phytobiotics Futterzusatzstoffe GmbH, 65343 Eltville (DE)
(72) Erfinder: ROTH, Hermann, 65346 Eltville (DE)
(74) Vertreter: Richardt Patentanwälte PartG mbB

(57) **Zusammenfassung**

Die Erfindung betrifft eine Substanzkombination zur Behandlung von Kokzidiose bei Nutztieren oder Hobbytieren, die Isochinolinalkaloide und ein Kokzidiostatikum oder ein Kokzidioseimpfmittel enthält.

## Beschreibung

### Technisches Gebiet

Die Erfindung bezieht sich auf eine Substanzkombination zur Behandlung von Kokzidiose bei Nutztieren.

### Stand der Technik

Als Kokzidiose bezeichnet man Erkrankungen durch Einzeller, die sogenannten Kokzidien (Klasse: Sporozoen). Kokzidiose gehört zu den Protozoeninfektionen. Die Kokzidien befallen vor allem den Darm, bei einigen Tieren auch Leber und Niere. Kokzidiosen spielen bei verschiedenen Haustieren eine große Rolle, insbesondere, aber nicht nur, als Jungtiererkrankung. Wirtschaftlich bedeutsam sind insbesondere die Kokzidiose des Rindes, des Schafes und des Geflügels, einschließlich der Hühnervögel. Die Krankheit tritt aber auch bei Kaninchen, Hasen, Hunden, Katzen, Ziegen und Fischen auf. Kokzidiose wird unter anderem von verschiedenen Eimeria-Arten hervorgerufen. Eimerieninfektionen sind besonders schädlich für die Geflügelindustrie und kosten z.B. die Vereinigten Staaten jährlich mehr als 1,5 Milliarden Dollar an Verlusten.

Kokzidiose wird heute durch Antibiotika, insbesondere ionophore Antibiotika, und duch eine Reihe nicht-ionophorer Kokzidiostatika behandelt. Teilweise werden die Tiere auch gegen Kokzidiose geimpft.

Allerdings treten oftmals nach wenigen Erreger-Generationen und/oder bereits nach kurzzeitiger Verwendung eines einzelnen Wirkstoffs Resistenzen auf, die die Wirksamkeit der eingesetzten Antibiotika erheblich reduzieren bis hin zur völligen Unwirksamkeit. Um dieses Problem zu umgehen, wurden die Antibiotika oft im Rotationsprinzip verabreicht, um Erregern mit Resistenzen gegen ein bestimmtes Antibiotikum nach Möglichkeit durch frühzeitigen Wechsel auf ein anderes Antibiotikum beizukommen. Insbesondere bei Hähnchenmastbetrieben mit hoher Besatzdichte und mehr Herdenzyklen pro Jahr in einer einzigen Anlage ist das schnelle Auftreten von Resistenzen ein großes Problem.

Verschärfend kommt hinzu, dass das Spektrum verfügbarer Antibiotika kleiner wird, teils, weil Resistenzen gegen bestimmte Antibiotika ubiquitär geworden sind, teils, weil gesetzliche Bestimmungen die Verwendung von Antibiotika wegen Rückstandbedenken oder Kreuzresistenzen zu Humanmedizinischen Antibiotika einschränken. Insbesondere der Einsatz ionophorer Antibiotika wird in vielen Ländern zunehmend stark vom Gesetzgeber eingeschränkt, um das Auftreten multiresistenter Erreger zu verhindern.

Nutztierhalter sehen sich zunehmend mit dem Problem konfrontiert, mit einem kleiner werdenden Spektrum verfügbarer Antibiotika dennoch ihren Tierbestand wirksam und ohne erhebliche Mehrkosten vor Kokzidiose zu schützen.

### Technisches Problem und grundlegende Lösungen

Der Erfindung liegt demgemäß die Aufgabe zugrunde, Alternativen zu den oben beschriebenen Verfahren zum Schutz von Nutztieren oder Hobbytieren vor Kokzidiose zur Verfügung zu stellen.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung eine Substanzkombination zur Behandlung von Kokzidiose bei Nutztieren oder Hobbytieren. Die Substanzkombination enthält Isochinolinalkaloide und enthält zudem ein Kokzidiostatikum oder ein Kokzidioseimpfmittel.

Der Begriff "Behandlung" ist hier breit zu verstehen, also im Sinne einer vorbeugenden Behandlung (Verabreichung bevor Krankheitssymptome beobachtet wurden) und/oder einer Behandlung nachdem bereits Symptome von Kokzidiose beobachtet wurden ("Akutbehandlung", Behandlung einer akuten Kokzidiose).

Ausführungsformen der Erfindung können den Vorteil haben, dass durch die Kombination eines Kokzidiostatikums oder Kokzidioseimpfmittels mit Isochinolinalkaloiden die Wirksamkeit des Kokzidiostatikums oder Kokzidioseimpfmittels deutlich erhöht werden kann. Dies bedeutet, dass bereits geringere Konzentrationen des Kokzidiostatikums ausreichen können, um Kokzidiose wirksam vorzubeugen oder diese nach Ausbruch zu bekämpfen. Beispielsweise wurde beobachtet, dass Ausführungsformen mit einer Kombination aus Isochinolinalkaloiden und einem Kokzidiostatikum oder einem Kokzidioseimpfmittel einen positiven synergistischen Effekt im Hinblick auf die Reduktion von kokzidiosebedingten Krankheitssymptomen wie auch der kokzidiosebedingten Mortalität haben können. Ausführungsformen können weiterhin den Vorteil haben, dass der Ausbreitung von resistenten Erregerstämmen entgegengewirkt wird, und dem Ausbruch von Kokzidiose in großen Tierbeständen wirksamer vorgebeugt wird. Resistenzbildung beruht auf einzelnen, zufälligen Veränderungen des Erbguts, sogenannten Mutationen, die den Stoffwechsel eines Erregers so verändern, dass der Erreger weniger empfindlich auf auf ein spezielles Antibiotika reagiert. Da es sich bei der Mutationsentstehung um ein zufälliges Ereignis handelt, das durch den Selektionsdruck des Einsatzes eines speziellen gegen die Kokzidien wirksamen Wirkstoffs in seiner Ausbreitung gefördert wurde, erhöht sich das Risiko, dass eine Mutation auftritt die zu einer verringerten oder ausbleibenden Empfindlichkeit der Kokzidien gegen den Wirkstoff führt, mit der Anzahl der an Kokzidiose erkrankten Tiere eines Tierbestands und mit der Gesamtzahl an Kokzidioseerregern. Ein möglichst früh einsetzender und wirksamer Schutz gegen eine Verbreitung des Erregers in einem Tierbestand reduziert daher das statistische Risiko und damit die Geschwindigkeit der Resistenzerzeugung.

Ausführungsformen können weiterhin den Vorteil haben, dass dadurch Kosten reduziert werden können: Isochinolinalkaloide können vergleichsweise günstig aus Pflanzenextrakten gewonnen werden, wohingegen viele Antibiotika chemisch oder biotechnologisch unter Laborbedingungen erzeugt werden müssen und daher in der Regel deutlich teurer sind. Dadurch, dass Isochinolinalkaloide die Wirksamkeit von Kokzdiostatika erhöhen, reduzieren sie den Verbrauch an Kokzidiostika , denn es kann für den gleichen Schutzeffekt weniger von dem teuren Antibiotkum eingesetzt werden und/oder es kann beim Einsatz einer gegebenen Menge Antibiotikum eine bessere Schutzwirkung erzielt werden, sodass das Risko des Befalls weiterer Tiere und der damit einhergehenden Antibiotikaeinsatz vermieden werden kann.

Nach Ausführungsformen beinhaltet die Substanzmsichung isochinolinhaltiges Pflanzenmaterial. Das für Ausführungsformen beschriebene Pflanzenmaterial kann z.B. ein pflanzlicher Extrakt sein. Ein pflanzlicher Extrakt kann beispielsweise wie nachfolgend beschrieben hergestellt werden: getrocknetes und pulverisiertes isochinolinhaltiges Pflanzenmaterial (z.B. pulverisierte Blätter oder Wurzeln) wird einer zweifachen hydroalkoholischen Extraktion (70% Ethanol) unterzogen. Das Lösungsmittel wird mittels Vakuumdestillation bei ca. 55-60°C entfernt. Dabei wird ein konzentrierter Extrakt gewonnen. Dieser Extrakt wird mit Maltodextrin versetzt, der resultierende flüssige Extrakt bei ca. 80-90°C vakuumgetrocknet und anschließend gemahlen.

Nach Ausführungsformen der Erfindung dient die Substanzkombination insbesondere zur Behandlung von kokzidieninduzierten Darmentzündungen bei Nutztieren oder Hobbytieren und/oder zur Behandlung von kokzidieninduzierten Durchfällen bei Nutztieren oder Hobbytieren.

Nach Ausführungsformen der Erfindung ist die Behandlung eine Vorbeugung, also eine Behandlung, bevor die Kokzidiose erkennbar ausgebrochen ist. Eine vorbeugende Behandlung kann eine Impfung sein.

Nach anderen Ausführungsformen der Erfindung ist die Behandlung eine Akutbehandlung, also eine Behandlung einer bereits ausgebrochenen Kokzidiose.

Bei der Substanzkombination kann es sich z.B. um ein Medikament, eine Tiernahrung, ein Tränkwasser, oder ein Tiernahrungsergänzungsmittel handeln.

Die Verwendung von Tiernahrungsergänzungsmitteln, die z.B. als flüssiges oder festes Konzentrat realisiert sein können, kann vorteilhaft sein, da die Alkaloide und das Kokzidiostatikum bzw. der Impfstoff darin homogen durchmischt vorliegen können. Der größte Volumenanteil des Tiernahrungsergänzungsmittels kann aber aus anderen Substanzen, z.B. Wasser, Milch, Pflanzenmaterialien, Soyamehl, Mais oder Maismehl oder Getreide bestehen. Wird ein solches Tiernahrungsergänzungsmittel mit Tränkwasser oder der eigentlichen Tiernahrung vermischt, kann dies die homogene Verteilug der bereits in geringen Mengen physiologisch Wirksamen Substanzen im Vergleich einer direkten Zugabe von Alkaloiden, Kokzidiostatika oder Impfstoffen erleichtern.

Durchfall ist eine häufige Folge von Kokzidiose und führt aufgrund der schlechteren Resorption von Nähr- und Mineralstoffen zu einer Wachstumsverzögerung bei Nutztieren oder Hobbytieren, einem allgemein verschlechterten Gesundheitszustand und in vielen Fällen auch zum Tod. Wenn erste Symptome erkennbar werden, ist es oftmals schon zu spät, um eine Ausbreitung der Kokzidiose im Tierbestand zu verhindern oder erfordert hohe Dosen eines Kokzidiostatikums.

Bezüglich einer Kombination aus Isochinolinalkaloiden und Kokzidostatika oder Kokzidioseimpfmittel hat der Anmelder beobachtet, dass diese kokzidieninduzierte Darmentzündungen und/oder Durchfall wirksamer bekämpft bzw. diesen vorbeugt als der Einsatz von Kokzidostatika oder Kokzidioseimpfmitteln alleine. Zusätzlich wird in vielen Fällen eine bessere Verdauung des Futters, eine bessere Futterausnutzung, eine höhere Eiweißausnutzung, und verbesserte Masttagzunahme oder Milchleistung bei gegebenem Einsatz von Futter bewirkt. Auch konnte eine ganz allgemein verbesserte Immunabwehr beobachtet werden, da der Darm für einen Großteil der Immunreaktion eines Körpers verantwortlich ist.

Nach Ausführungsformen der Erfindung wird die Substanzkombination über die gesamte Mast hinweg (täglich oder regelmäßig im Abstand von einigen wenigen Tagen) verabreicht. Vorzugsweise wird die Verabreichung mindestens fünf Tage vor Ende der Mast beendet.

Nach Ausführungsformen der Erfindung handelt es sich bei dem Kokzidiostatikum um ein ionophores Antibiotikum.

Unter einem "Ionophor" wird hier ein Molekül verstanden, das Ionen durch eine Membran transportiert. Einige Bakterien bilden lonophore, also Moleküle, die die Permeabilität von Membranen für Ionen erhöhen. Oft haben solche Moleküle eine antibiotische Wirkung, da sie lonenkonzentrationsgefälle abbauen. Ein lonophor mit antibiotischer Wirkung wird auch als ionophores Antibiotikum bezeichnet. Manche lonophore werden von Bakterien als porenbildendes Toxin verwendet. Beispielsweise sing sog. "Carrier-Ionophore" und "kanalbildende lonophore" bekannt.

Nach Ausführungsformen der Erfindung ist das ionophore Antibiotikum Salinomycin, Monensin oder Narasin.

Dies kann vorteilhaft sein, da die besagten Antbiotika in vielen Ländern zugelassen sind, sodass viele Nutztierhalter mit der Handhabung und Dosierung dieser Antibiotika bereits vertraut sind.

Nach anderen Ausführungsformen der Erfindung handelt es sich bei dem Kokzidiostatikum um ein nicht-ionophores Antibiotikum. Vorzugsweise handelt es sich bei dem Kokzidiostatikum um ein nicht-ionophores Antibiotikum mit fehlender oder nur schwacher antibakterieller Wirkung, insbesonde um reine Antiprotozoika.

Nicht-ionophore Antibiotika, insbesondere nicht-ionophore Antibiotika, die nicht oder nur geringfügig anitbakteriell wirken, können den Vorteil haben, dass sie deutlich sicherer im Einsatz sind und eine viel geringere Toxizität für das Tier haben als z B. das Narasin oder das Salinomycin. So ist der Faktor von Einsatzdoiserung zu toxischer Dosierung beim z B. Diclazuril eins zu 100 während die toxische Dosis beim Salinomycin bereits beim nur 5-fachen der Einsatzsdosierung liegt. Zudem haben nicht-ionophore Kokzisiostatika alle den Nachteil einer sehr raschen und häufig spontan auftretenden Resistenzbildung bis hin zu rascher vollkommener Resistenz und damit völligem Wirkungsverlust, die bei lonophoren Kokzidiostatika nie derart auftritt. weniger als ionophore Antibiotika die Ausbildung von Resistenzen fördern. Nicht-ionophore Kokzidiostika wie Diclazuril oder Nicarbazin sind daher eigentlich in der Kokzidiosevorbeuge zu bevorzugen, sie sind kaum toxisch, sie sind nicht antibakteriell wirksam sondern nur kokzidienwirksam und selektieren keine resistenten Bakterien (Kokzidien sind keine Bakterien), sie sind umweltfreundlicher, nachhaltiger und auch im Hinblick auf die Gefahr multiresistenter Erreger für die menschliche Gesundheit weniger problematisch.

Nach Ausführungsformen der Erfindung ist das nicht-ionophore Antibiotikum Nicarbazin, Amprolium, Diclazuril, Toltrazuril oder Halofuginon. Nach bevorzugten Ausführungsformen der Erfindung ist das nicht-ionophore Antibiotikum Nicarbazin.

Beispielsweise wird derzeit das Medikament Maxiban, eine Kombination aus Nicarbazin mit dem ionophoren Antibiotikum Narasin, als Kombipräparat gegen Kokzidiose eingesetzt, wobei allerdings Narasin den Nachteil hat, dess es wegen seines antibakteriellen Charakters zu gesetzgeberischen Restriktionen in immer weniger Ländern zum Einsatz kommt. Daher, und auch um dem Kundenwunsch nach medizinisch unbedenklichen Behandlungsoptionen zu folgen, suchen Geflügelmäster nach Alternativen. Ein Ersatz des Antibiotikums Narasin durch die Isoquinolinalkaloide in solchen Kombipräparaten gemäß Ausführungsformen der Erfindung kann die besagten Nachteile von ionophoren Antibiotika überwinden, da Isochinolinalkaloide nicht dazu neigen, die Entstehung von resistenten Erregern zu induzieren.

Nach Ausführungsformen der Erfindung hat das nicht-ionophore Antibiotikum keine oder nur eine schwache antibakterielle Wirkung. Dies kann den Vorteil haben, dass die Bildung von resistenten Bakterienstämmen, die aufgrund des genetischen Austauschs mit pathogenen Bakterienstämmen, die den Menschen befallen können, vermieden wird. Beispielsweise sind die nicht-ionophoren Antibiotika Nicarbazin, Amprolium, Diclazuril, Toltrazuril und Halofuginon nicht oder nur schwach antibakteriell. Sie wirken im Wesentlichen antiprotozoisch.

Nach anderen Ausführungsformen der Erfindung handelt es sich bei dem Kokzidiostatikum um ein nicht-ionophores Antibiotikum und die Tiernahrung, das Tränkwasser und/oder das Tiernahrungsergänzungsmittel ist frei von ionophoren Antibiotika.

Nach Ausführungsformen der Erfindung enthält die Substanzkombination das Nicarbazin in einer Konzentration von 20 mg bis 500 mg, vorzugsweise von 50 mg bis 200 mg pro kg Tiernahrung.

Nach anderen Ausführungsformen der Erfindung ist die Substanzkombination eine Tiernahrung, welche das Nicarbazin in einer Konzentration von 20 mg bis 500 mg, vorzugsweise von 50 mg bis 200 mg pro kg Tiernahrung enthält.

Nach anderen Ausführungsformen der Erfindung ist die Substanzkombination ein Tränkwasser, welches das Nicarbazin in einer Konzentration von 20 mg bis 500 mg, vorzugsweise von 50 mg bis 200 mg pro Liter Tränkwasser enthält.

Nach anderen Ausführungsformen der Erfindung ist die Substanzkombination ein Medikament oder ein Tiernahrungsergänzungsmittel, welches das Nicarbazin in einer Konzentration enthält, die eine bestimmungsmäßige Beimischung des Medikaments oder des Tiernahrungsergänzungsmittels zu einer Tiernahrung eine Tiernahrung mit einer Nicarbazinkonzentration von 20 mg bis 500 mg, vorzugsweise von 50 mg bis 200 mg pro kg Tiernahrung ergibt.

Nach anderen Ausführungsformen der Erfindung ist die Substanzkombination ein Medikament oder ein Tiernahrungsergänzungsmittel, welches das Nicarbazin in einer Konzentration enthält, die eine bestimmungsmäßige Beimischung des Medikaments oder des Tiernahrungsergänzungsmittels zu Tränkwasser ein Tränkwasser mit einer Nicarbazinkonzentration von 20 mg bis 500 mg, vorzugsweise von 50 mg bis 200 mg, pro Liter Tränkwasser ergibt.

Nach Ausführungsformen wird die Substanzkombination mit den Isochinolinalkaloiden und dem Nicarbazin zur Vorbeugung und/oder Behandlung von Zäkum- und Dünndarmkokzidiose bei Hühnern verwendet. Die oben genannten Konzentrationsbereiche können vorteilhaft sein, da diese sicherstellen, dass ein Nutztier im Rahmen der üblicherweise aufgenommenen Mengen an Futtermitteln oder Tränkwasser eine physiologisch wirksame Menge des Nicarbazins aufnimmt, wobei die aufgenommene Konzentration nicht so hoch ist, dass hierdurch unerwünschte und inakzeptable Nebenwirkungen auftreten.

Die Verwendung von Nicarbazin kann vorteilhaft sein, da Nicarbazin gegen mehrere Arten von Eimeria aktiv ist. Im Gegensatz zu anderen Kokzidiostatika, insbesondere ionophoren Antibiotika, induziert Nicarbazin nur selten Resistenzerscheinungen in den häufigsten Kokzidioseerregern, wie z.B. in Eimeria tenella.

Die Verwendung einer Kombination von ionophoren oder nicht-ionophoren Antibiotika mit Isochinolinalkaloiden kann vorteilhaft sein, da das Wirkungsspektrum dieser Kokzidostatika erhöht und die Stärke und Geschwindigkeit der Resistenzbildung verringert wird.

Nach anderen Ausführungsformen der Erfindung beinhaltet die Substanzkombination ein Kokzidioseimpfmittel anstelle eines Kokzidiostatikums. Bei dem Kokzidioseimpfmittel handelt es sich z.B. um einen Lebendimpfstoff, der sporulierte Oozysten von einer oder mehreren Eimeria-Arten beinhaltet.

Die Substanzkombination mit dem Kokzidioseimpfmittel wird vorzugsweise prophylaktisch verabreicht, also bevor aufgrund von beobachteten Krankheitssymptomen und/oder veterinärmedizinischen Untersuchungen bereits der Ausbruch der Krankheit Kokzidiose in einigen oder allen Tieren eines Tierbestands sicher festgestellt wurde. Die prophylaktische Verabreichung soll von vorneheirein verhindern, dass Kokzidiose entsteht oder, falls sie latent schon in einigen Tieren vorhanden sein sollte, sich im Tierbestand verbreitet.

Vorzugsweise beinhaltet das Impfmittel Erregerarten, z.B. Eimeriaarten, die unter Berücksichtigung der Nutztierart bzw. Hobbytierart und/oder unter Berücksichtigung bisheriger bekannter Infektionen mit dieser Erregerart ausgewählt wurden.

Die Verwendung eines Impfmittels kann vorteilhaft sein, da eine Impfung bewirkt, dass das geimpfte Tier auch nach Absetzen des Impfmittels vor einer Kokzidiose geschützt ist. Dies beruht darauf, dass bei einer Impfung das Immunsystem des Tieres gelernt hat, den Kokzidioseerrekter zu erkennen und effektiv zu bekämpfen.

Die Resistenzproblematik bei Kokzidostatika, insbesondere ionophoren Kokzidostatika, hat die Tierhalter oftmals dazu gezwungen, mehrere Anitbiotika im Wechsel einzusetzen, bis die Tiere das Schlachtalter erreicht haben. Oftmals war der Zeitraum zwischen dem ersten Auftreten von Krankheitssymptomen und der massiven Verbreitung innerhalb eines Tierbestands so kurz, dass eine Ausbreitung der Kokzidiose nicht mehr oder nur unter hohem Antibiotikaeinsatz verhindert werden konnte. Die Verwendung von Impfmitteln dagegen kann den Vorteil haben, dass der Tierhalter den Anstieg einer ggf. nur subklinisch manifestierten Kokzidiose nicht mehr ganz so engmaschig kontrollieren muss, da die Tiere ja geimpft sind. Als besonders wirksam haben sich Impfungen mit Lebendimpfstoff erwiesen. Die Zeit von der Impfung bis zur vollständigen Immunität beträgt bei Masthähnchen z.B. oftmals 21 bis 28 Tage, je nach den Impfstoff- und Haltungsbedingungen. Dieser lange Zeitraum ist von erheblichem Nachteil und schränkt die Verwendbarkeit der Impfung zur Kokzisiosevorbeuge ein, da gerade im Zeitraum vom 14. bis 21. Lebenstag der erste hohe Infektionsdruck der Kokzidien stattfinmdet, dem Zeitraum, in dem die Impfung noch nicht ihre volle Wirksamkeit entfaltet hat. Isochinolin Alkaloide können diesen kritischen Zeitraum der Infektion durch Kokzidien wirkdungsvoll überbrücken helfen. Ein wesentlicher Vorteil kann auch darin liegen, dass bei einer Impfung keine Resistenzbildung des Erregers auftritt. Eine Impfung ist eine nachhaltige Maßnahme, um sowohl klinische als auch subklinische Kokzidiosen zu vermeiden. Eine Impfung kann ein Fortschreiten der Kokzidiose auf einem subklinischen Niveau wirksam verhindern, was beim Einsatz von Kokzidiostatika nicht immer gelingt, da eine subklinische Ausprägung und Ausbreitung der Krankheit schwer zu erkennen ist. Dies kann für die Tierhalter zu erheblichen wirtschaftlichen Nachteilen führen: Durch subklinische Kokzidiose hervorgerufene niedriggradige Darmläsionen können spät im Herdenzyklus auftreten und sich nachteilig auf die Gewichtszunahme, die Tage bis zur Vermarktung, die Futterverwertung und den Fleischertrag auswirken.

Entsprechende Impfprodukte sind auf dem Markt erhältlich, z.B. FORTEGRA® vom Hersteller MSD Animal Health (in den Vereinigten Staaten und Kanada als Merck Animal Health bekannt). FORTEGRA enthält die Kokzidiose-Stämme, die in Masthähnchenställen vorkommen, einschließlich frühreifer und klassischer Stämme von Eimeria (E.) maxima, und führt zu einer schnelleren Immunität und einem breiten Schutz. FORTEGRA ist wirksam bei der Bekämpfung aller relevanten Kokzidiose-Stämme in Masthähnchen und -hühnern, einschließlich E. acervulina, E. mivati, E. maxima und E. tenella. Der Impfstoff setzt die Herde gleichzeitig zwei Stämmen der Kokzidioseart E. maxima aus, von denen einer ein frühreifer Stamm ist, der eine frühere und stärkere Immunität stimuliert. Eine frühere Immunität lässt mehr Zeit für kompensatorisches Wachstum und kann Leistungsverluste verhindern.

Nach Ausführungsformen der Erfindung enthält die Substanzkombination Pflanzenmaterial der Gattung Sanguinaria (insbesondere Sanguinaria canadensis), und/oder Pflanzenmaterial der Gattung Argemone (insbesondere Argemone mexicana), und/oder Pflanzenmaterial der Gattung Chelidonium (insbesondere Chelidonium majus), und/oder Pflanzenmaterial der Gattung Macleaya (insbesondere Macleaya cordata), oder eines Extraktes eines oder mehrerer dieser Pflanzenmaterialien, wobei die Isochinolinalkaloide Bestandteil des Pflanzenmaterials sind.

Nach Ausführungsformen der Erfindung sind die Isochinolinalkaloide Benzophenanthiridin-Alkaloide in physiologisch wirksamer Menge. Die Isochinolinalkaloide können insbesondere das Sanguinarin, das Chelerythrin oder eine Mischung dieser beiden Isochinolinalkaloide sein.

Gemäß einem Beispiel beinhalten die Isochinolinalkaloide eines Sanguinaria Canadensis Extrakts: 35 bis 55 Gew.-% Sanguinarin, 20 bis 30 Gew.-% Chelerythrin, und eine Mischung weiterer Isochinolinalkaloide, wie z.B. Chelirubin. In einem anderen Beispiel wurde ein Sanguinaria Canadensis Extrakt verwendet, bei welchem Sanguinarin und Chelerythrin jeweils einen Anteil von über 40% der Isochinolinalkaloide des Extrakts ausmachten. In manchen Ausführungsformen können auch annähernd 100% der Isochinolinalkaloidie des Extrakts aus Sanguinarin und/oder Chelerythrin bestehen. Bei dem Extrakt kann es sich z.B. um einen alkohloischen Extrakt handeln.

In anderen Ausführungsformen werden die Isochinolinalkaloide der Substanzkombination als einzelne chemische Substanz, die durch chemische oder biotechnologische Synthese oder durch substanzspezifische Extraktion aus Pflanzenmaterial gewonnen wurde, individuell zugesetzt.

Die Alkaloide können in der Substanzkombination z.B. frei vorkommen, oder als physiologisch akzeptable Salze, z.B. als Sanguinarinchlorid.

Nach Ausführungsformen der Erfindung enthält die Substanzkombination die Isochinolinalkaloide in einer Konzentration von 0,1 mg bis 10 mg, vorzugsweise von 0,5 mg bis 5 mg pro kg Substanzkombination.

Nach Ausführungsformen der Erfindung ist die Substanzkombination eine Tiernahrung oder ein Tränkwasser. Die Substanzkombination enthält die Isochinolinalkaloide in einer Konzentration von 0,1 mg bis 10 mg, vorzugsweise von 0,5 mg bis 5 mg pro kg Substanzkombination.

Nach einer weiteren Ausführungsform ist die Substanzkombination ein Tiernahrungsergänzungsmittel oder Medikament, welches die Isochinolinalkaloide in einer Konzentration enthält, die bei bestimmungsmäßiger Beimischung der Substanzkombination in eine Tiernahrung eine Tiernahrung mit einer Isochinolinalkaloidkonzentration von 0,1 mg bis 10 mg, vorzugsweise von 0,5 mg bis 5 mg pro kg Tiernahrung ergibt.

Nach einer weiteren Ausführungsform ist die Substanzkombination ein Tiernahrungsergänzungsmittel oder Medikament, welches die Isochinolinalkaloide in einer Konzentration enthält, die bei bestimmungsmäßiger Beimischung der Substanzkombination in ein Tränkwasser ein Tränkwasser mit einer Isochinolinalkaloidkonzentration von 0,1 mg bis 10 mg, vorzugsweise von 0,5 mg bis 5 mg pro Liter Tränkwasser ergibt.

Nach Ausführungsformen der Erfindung handelt es sich bei den Nutztieren um Rinder, Kühe, Schweine, Pferde, Geflügel, Schafe, Ziegen, Kaninchen, oder Fische, und insbesondere um Geflügel, und/oder bei den Hobbytieren um Hunde oder Katzen.

Nach Ausführungsformen der Erfindung liegt die pro Tag verabreichte Menge an Isochinolinalkaloiden zwischen 0,02 und 3,0 mg pro kg Lebendgewicht der Nutztiere oder Hobbytiere, bevorzugt zwischen 0,02 und 1,0 mg pro kg Lebendgewicht der Nutztiere oder Hobbytiere.

Nach Ausführungsformen der Erfindung ist das verwendete Kokzidiostatikum Nicarbazin. Die pro Tag verabreichte Menge an Nicarbazin liegt bei mindestens 2 mg Nicarbazin pro kg Lebendgewicht der Nutztiere oder Hobbytiere, bevorzugt zwischen 2 und 50 mg Nicarbazin pro kg Lebendgewicht.

In einem weiteren Aspekt betrifft die Erfindung ein Verfahren zur Vorbeugung und/oder Behandlung von Kokzidiose bei Nutztieren oder Hobbytieren. Das Verfahren ist dadurch gekennzeichnet, dass es ein Füttern der Substanzkombination nach Ausführungsformen der Erfindung an Nutztiere umfasst.

Die oben genannten Konzentrationsbereiche können vorteilhaft sein, da diese sicherstellen, dass ein Nutztier im Rahmen der üblicherweise aufgenommenen Mengen an Futtermitteln oder Tränkwasser eine physiologisch wirksame Menge der Isochinolinalkaloide aufnimmt, wobei die aufgenommene Konzentration nicht so hoch ist, dass hier durch unerwünschte und inakzeptable Nebenwirkungen auftreten.

In einem weiteren Aspekt betrifft die Erfindung eine Verwendung einer Substanzkombination nach einer der hier beschriebenen Ausführungsformen zur Vorbeugung und/oder Behandlung von Kokzidiose bei Nutztieren oder Hobbytieren.

Nach Ausführungsformen sind die Isochinolinalkaloide Benzophenanthridinalkaloide, vorzugsweise ausgewählt aus der Gruppe bestehend aus Sanguinarin und/oder Chelerythrin und/oder deren Salzen.

Dies kann vorteilhaft sein, da alkaloidhaltige Pflanzenextrakte kostengünstig herstellbar sind und keine Antibiotikaresistenzen verursachen.

Isochinolinalkaloide sind biologisch abbaubar und können als Naturprodukt (Pflanzenextrakt) in vergleichsweise großen Mengen gewonnen werden. Dies entspricht auch den Wünschen des Konsumenten nach einem naturbelassenen Lebensmittel und einer nachhaltigen, umweltfreundlichen und im Hinblick auf die Entwicklung multiresistenter Erreger unbedenklichen Lebensmittelproduktion.

Somit kann in manchen Fällen auf den Einsatz von ionophoren Antibiotika, die in manchen Ländern verboten sind, verzichtet werden, oder der Einsatz von solchen Antibiotika kann reduziert werden.

Ein Medikament oder Tiernahrungsergänzungsmittel nach einem der hier beschriebenen Ausführungsformen kann zur Erzeugung der Tiernahrung bzw. des Tränkwassers nach einem der hierin beschriebenen Ausführungsformen verwendet werden. Das Tiernahrungsergänzungsmittel kann beispielsweise eine (Tiernahrungs)vormischung oder ein Konzentrat sein, das sowohl Isochinolinalkaloide als auch ein Kokzidiostatikum oder ein Kokzidioseimpfmittel enthält. Beispielsweise können Dosierempfehlungen auf der Verpackung des Tiernahrungsergänzungsmittels so lauten, dass bei empfehlungsgemäßer Dosierung eine Tiernahrung bzw. ein Tränkwasser nach den hier beschriebenen Ausführungsformen beim Endabnehmer erzeugt wird.

In einem weiteren Aspekt betrifft die Erfindung ein Verfahren zur Behandlung von Nutztieren oder Hobbytieren gegen Kokzidiose, wobei das Verfahren die Bereitstellung (Verfütterung) einer Substanzkombination nach einer der hier beschriebenen Ausführungsformen an Nutztiere oder Hobbytiere beinhaltet. Die Bereitstellung kann z.B. in einem Befüllen eines Futtertrogs oder eines Futterautomaten mit entsprechender Tiernahrung oder dem Befüllen einer Tränkanlage oder eines Wassertrogs mit einem entsprechenden Tränkwasser oder sonstiger Formen der Bereitstellung der erfindungsgemäßen Substanzkombination an Nutztiere oder Hobbytiere bestehen. Die Substanzkombination kann fest oder flüssig sein. So wird hier z.B. auch die sogenannte "Kälbermilch" als Tiernahrung verstanden, welche die genannte Kombination von einem oder mehreren Isochinolinalkaloiden einerseits und einem Kokzidiostatikum oder einem Kokzidioseimpfmittel andererseits beinhaltet. Die Zusammensetzung und Darreichungsform kann in Abhängigkeit von der Tierart (z.B. Geflügel, Rinder, Schweine, Schafe, Ziegen, Kaninchen, Hund, Katze etc.), dem Alter der Tiere und dem Verfütterungsort (Stall, Freiland, etc.) variieren.

Optional kann die Substanzkombination weitere Zusatzstoffe wie Vitamine, Spurenelemente, Aminosäuren, Kräuter, Geschmacksstoffe etc. beinhalten. Das Isochinolinalkaloid kann der Substanzkombination in Form eines Pflanzenextrakts zugegeben werden, welcher z.B. Alkaloide wie Sanguinarin und/oder Chelerythrin enthält.

Nach Ausführungsformen der Erfindung ist die Substanzkombination als Substanzmischung, insbesondere als homogen gemischte flüssige oder feste Substanzmischung, ausgebildet. Beispielsweise kann die Substanzkombination ein Medikament, eine Tiernahrung, ein Tiernahrungsergänzungsmittel, oder ein Tränkwasser sein, welches bereits sowohl die Isochinolinalkaloide als auch das Kokzidiostatikum oder das Kokzidioseimpfmittel beinhaltet.

Nach anderen Ausführungsformen ist die Substanzkombination als Behandlungsset ausgebildet. Das Behandlungsset beinhaltet mindestens zwei Komponenten, wobei eine der Komponenten die Isochinolinalkaloide und die andere Komponente das Kokzidiostatikum oder das Kokzidioseimpfmittel enthält. Eine Komponente kann z.B. als Flasche, Glasampulle, Tube oder sonstiges portables Behältnis ausgebildet sein, sodass sich die einzelnen Komponenten unabhängig voneinander bewegen und handhaben lassen. Beispielsweise kann ein Behandlungsset aus zwei Behältnissen bestehen, wobei das eine Behältnis Tiernahrungszusatzstoffe einschließlich der Isochinolinalkaloide beinhaltet und das andere Behältnis eine kleine Glasampulle oder kleines Glasbehältnis mit einem Kokzidioseimpfstoff. Dies kann z.B. die Lagerung vereinfachen, da einzelne Komponenten, insbesondere die Komponente mit einem Kokzidioseimpfmittel, gekühlt oder besonders geschützt aufbewart werden können, was für die Lagerung der Isochinolinalkaloid-Komponente in der Regel nicht erforderlich ist.

Unter **"Nutztieren"** wird hier jegliche Tierart verstanden, die von Menschen aus kommerziellen Gesichtspunkten gehalten, gemästet und/oder vermehrt wird, insbesondere von landlebenden Säugetieren (insb. Schwein, Rind, Ziege und Schaf) und Geflügel, aber auch Fische.

Unter "Hobbytieren" werden hier Haustiere verstanden sowie Tiere, die für sonstige Zwecke von Menschen gehalten werden, z.B. zum Zwecke des Sports oder in Zoos und Aquarium für edukative Zwecke. Zu den Hobbytieren gehören z.B. Hund und Katze oder Kaninchen, Meerschweinchen etc., die z.B. im Haushalt gehalten werden und die alle ebenfalls an Kokzidien erkranken können.

Unter einer **"Kokzidiose"** wird hier eine Erkrankungen durch Einzeller, die sogenannten Kokzidien (Klasse: Sporozoen), verstanden. Sie gehören zu den Protozoeninfektionen. Die Kokzidien befallen vor allem den Darm, bei einigen Tieren auch Leber und Niere. Die Kokzidiose der Hühnervögel ist eine häufige parasitäre Erkrankung der Hühnervögel. Sie wird durch bestimmte Einzeller (Protozoen), den sogenannten Kokzidien verursacht, die der Gattung Eimeria angehören. Die Kokzidiosen der Hühnervögel sind vornehmlich Darmkokzidiosen.

Unter einer **"Behandlung"** von Tieren wird hier eine Verabreichung zumindest einer physiologisch gegen eine bestimmte Krankheit wirksamen Substanz an Tiere verstanden, nachdem, während, oder bevor diese Krankheit erkennbar in einem oder mehreren dieser Tiere ausgebrochen ist.

Unter einer **"Vorbeugung"** bzw. **"vorbeugender Behandlung"** wird hier eine Verabreichung von zumindest einer physiologisch gegen eine bestimmte Krankheit wirksamen Substanz an Tiere verstanden, bevor diese Krankheit erkennbar in einem oder mehreren dieser Tiere ausgebrochen ist. Eine vorbeugende Behandlung kann in einer Impfung oder in der vorbeugenden Verabreichung von akut wirkenden Substanzen, z.B. Kokzidiostatika, bestehen (die zwar keinen Impfeffekt haben, aber zumindest gegen latent schon vorhandene Erreger, die noch keine sichtbaren Symptome bewirkt haben, wirken kann).

Unter einer **"Akutbehandlung"** wird hier eine Verabreichung von zumindest einer physiologisch gegen eine bestimmte Krankheit wirksamen Substanz (z.B. Kokzidiostatika) an Tiere verstanden, nachdem diese Krankheit erkennbar in einem oder mehreren dieser Tiere ausgebrochen ist.

Unter einem **"Kokzidioseimpfmittel"** wird hier ein Impfmittel (auch "Vakzin") verstanden, das ein Tier vor der übertragbaren Krankheit Kokzidiose oder zumindest einigen Kokzidioseformen schützt. Ein Kokzidioseimpfmittel bewirkt eine Aktivierung des Immunsystems gegen spezifische Stoffe, die mit einer Infektion durch einen oder mehrere Kokzidioseerreger einhergehen. Impfungen wurden als vorbeugende Maßnahme gegen Infektionskrankheiten entwickelt. Eine Impfung befähigt das körpereigene Immunsystem auf eine Infektion mit dem Erreger so rasch und wirksam zu reagieren, dass daraus keine oder nur eine abgeschwächte Infektionskrankheit resultiert. Ein Impfmittel kann z.B. ein Lebendimpfstoff oder ein Totimpfstoff (z.B. Toxoidimpfstoffe) sein.

Unter einem **"Kokzidiostatikum"** wird hier ein Wirkstoff verstanden, der vorbeugend oder akut gegen den Befall von Tieren mit Kokzidien-Arten einsetzbar ist. Kokzidiostatika sind oftmals Antiprotozoenmittel, z.B. Amprolium, Arprinocid, Artemether, Clazuril, Clopidol, Decoquinat, Diclazuril, Dinitolmid, Ethopabate, Maduracmicin, Semduramicin, Halofuginon, Lasalocid, Monensin, Narasin, Nicarbazin, Oryzalin, Ponazuril, Robenidin, Roxarson, Salinomycin, Spiramycin, Sulfadiazin, Sulfadimethoxin, Toltrazuril. Gemäß Ausführungsformen besteht das Kokzidiostitikum aus einem oder mehreren der vorgenannten Kokzidiostatika.

Unter einem **"Antibiotikum"** wird hier eine teilsynthetische, vollsynthetische oder natürlich (.zB. durch Fermentation) gebildete Substanz, oder ein Substanzgemisch verstanden, welches schon in geringer Konzentration das Wachstum von Mikroorganismen hemmt oder diese abtötet. Bei den Mikroorganismen kann es sich um Bakterien, Protozoen, Pilze, oder Viren handeln. Insbesondere kann das Antibiotikum als Antiprotozoikum ausgebildet sein, z.B. als Kokzidiostatikum. Unter der Bezeichnung Antibiotikaresistenz werden Eigenschaften von Mikroorganismen wie Bakterien oder Pilze zusammengefasst, welche ihnen ermöglichen, die Wirkung von antibiotisch aktiven Substanzen abzuschwächen oder ganz zu neutralisieren.

### Kurzbeschreibung der Figuren

Einige Ergebnisse empirischer Tests bezüglich der Wirksamkeit einer Kombination aus Isochinolinalkaloiden einerseits und unterschiedlichen Klassen von Kokzidiostatika oder eines Kokzidioseimpfmittels andererseits und sind in den nachfolgenden Figuren veranschaulicht. Darin zeigen:
- Figur 1: ein Foto von 80 Masthähnchen unter ähnlichen Haltungsbedingungen wie die in dem Test untersuchten Tiergruppen,
- Figur 2: ein Foto einer Impfung eines Kükens,
- Figur 3: eine Tabelle mit Komponenten der verwendeten Futtermischungen während der Testlaufzeit,
- Figur 4: eine Tabelle mit Eimeria Läsionsscores der getesteten Masthähnchengruppen im Alter von 14 Tagen,
- Figur 5: eine Tabelle mit Eimeria Läsionsscores der getesteten Masthähnchengruppen im Alter von 28 Tagen,
- Figur 6: eine Tabelle mit Eimeria Läsionsscores der getesteten Masthähnchengruppen im Alter von 42 Tagen,
- Figur 7: eine Tabelle mit Sterblichkeiten der getesteten Masthähnchengruppen,
- Figur 8: eine Tabelle mit kumulierten Sterblichkeiten der getesteten Masthähnchengruppen,
- Figur 9: eine Tabelle mit Gewichtszunahmen verschiedener getesteter Masthähnchengruppen,
- Figur 10: eine Tabelle mit kumulierten Gewichtszunahmen der getesteten Masthähnchengruppen,
- Figur 11: eine Tabelle mit Futterkonversionsraten der getesteten Masthähnchengruppen,
- Figur 12: eine Tabelle mit kumulierten Futterkonversionsraten der getesteten Masthähnchengruppen,
- Figur 13: eine Tabelle mit Futteraufnahmemengen der getesteten Masthähnchengruppen,
- Figur 14: eine Tabelle mit kumulierten Futteraufnahmemengen der getesteten Masthähnchengruppen,

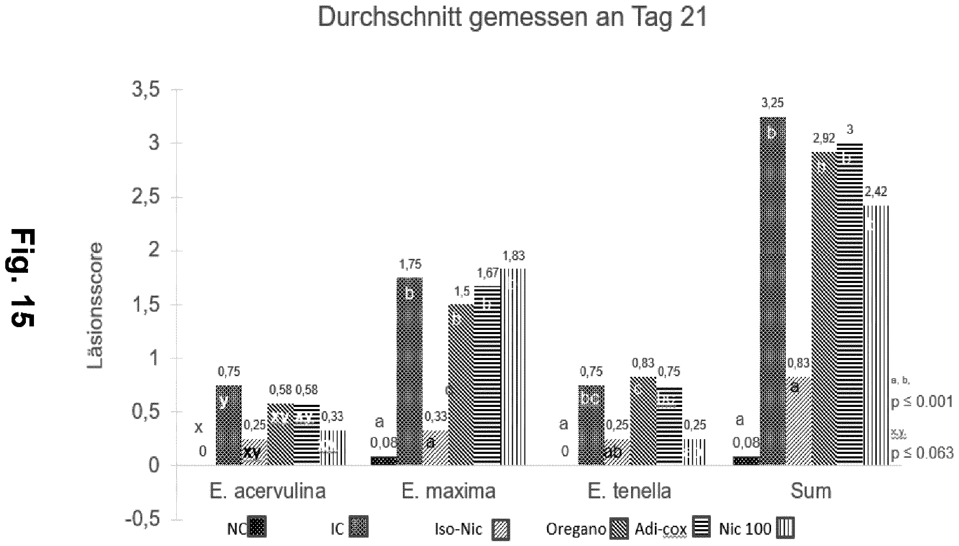

- Figur 15: die beobachteten Läsionsscores in Masthänchen in einer weiteren Wirksamkeitsstudie,
- Figur 16: die beobachtete Oocystenzahl in Ausscheidungen der Tiere der weiteren Studie, und
- Figur 17: die beobachtete Oocystenmasse in Ausscheidungen der Tiere der weiteren Studie.

### Ausführliche Beschreibung

Im Folgenden wird die Wirksamkeit von Substanzkombinationen gemäß verschiedenen Ausführungsformen der Erfindung gegen Kokzidiose bei Masthähnchen anhand experimenteller Daten veranschaulicht.

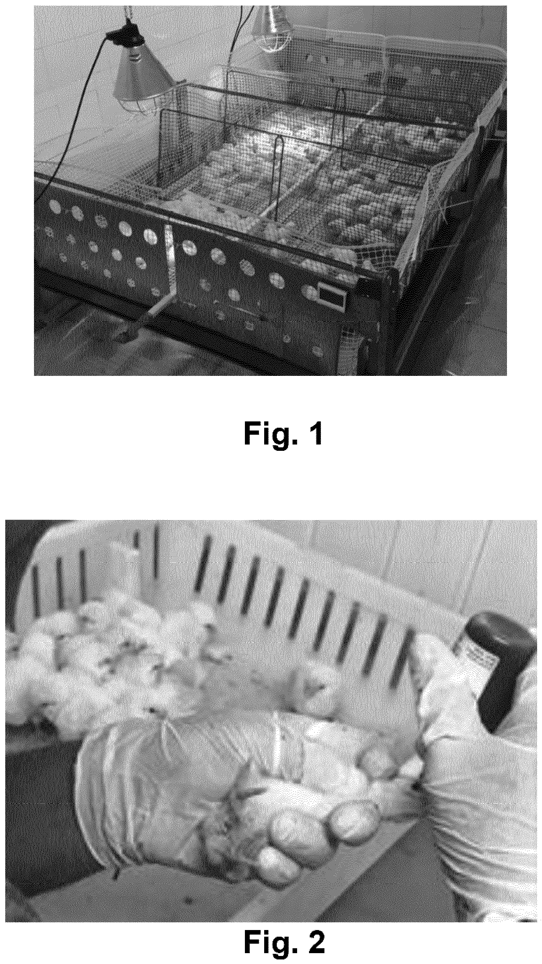

Figur 1 ein Foto von 80 Masthähnchen unter ähnlichen Haltungsbedingungen wie die in dem Test untersuchten Tiergruppen. Der Test hatte zum Ziel, den Effekt einer Kombination aus Isochinolinalkaloiden (Sanguinarin und Chelerythrin) mit bekannten Kokzidiostatika oder Kokzidioseimpfmitteln auf Kokzidien und die von diesen beeinträchtigte Darmgesundheit von Masthähnchen zu ermitteln. Es sollte gezeigt werden, ob der kombinierte Einsatz bekannter Anti-Kokzidiosemittel mit Isochinolinalkaloiden die Kontrolle von Kokzidieninfektionen verbessern kann.

Um dies herauszufinden, wurden die Masthähnchen Kokzidien-Oocysten ausgesetzt. Untersucht wurden mehrere kommerziell genutzte antikozidiale Behandlungsoptionen, bestehend aus einem nicht-ionophoren Kokzidiostatikum (Nicarbazin), einem ionophoren antimikrobiellen Kokzidiostatikum (Salinomycin) und einer Impfung. Da das ionophore Antibiotikum häufig Resistenzen induziert, und da auch die Impfung einige kritische Schwächen hat, die die Gesundheit der Tiere beeinflussen kann, sollte untersucht werden, ob die Nachteile der Monotherapien bzw. der Impfung als alleinige Maßnahme durch die Kombination mit den Isochinolin-Alkaloiden überwunden werden können.

Der Test wurde am 7. Februar 2019 auf Basis von 1.782 einem Tag alten männlichen "Cobb 500" Masthähnchen begonnen. Die Küken wurden nach dem Zufallsprinzip in 11 Tiergruppen aufgeteilt. Die Tiere der Tiergruppe wurden 10 unterschiedlichen Behandlungen unterzogen. Insgesamt wurden 99 Versuchsabteile mit jeweils 18 Tieren verwendet, was einer Dichte von 30 kg/m2 (6.14 lbs/ft2) am Ende der Studie entsprach. Die 99 Versuchsabteile sind in einer kommerziellen Versuchsabteilanlage von 14 x 130 m untergebracht. Jedes Versuchsabteil ist 1,7 x 1,7 m groß.

Im Zuge der Studie wurde eine experimentelle Infektion der Masthähnchen mit einer kommerziell erhältlichen Mischung von Lebendstämmen pathogener Kokzidiose Eimeria Erregern durchgeführt. Hierfür wurde jedem Küken am Tag 10 seines Lebens oral sporulierte Oozysten mehrerer Kokzidienarten verabreicht, nämlich je 35.000 Eimeria (E.) acervulina, je 10.000 E. maxima und 10.000 E. tenella.

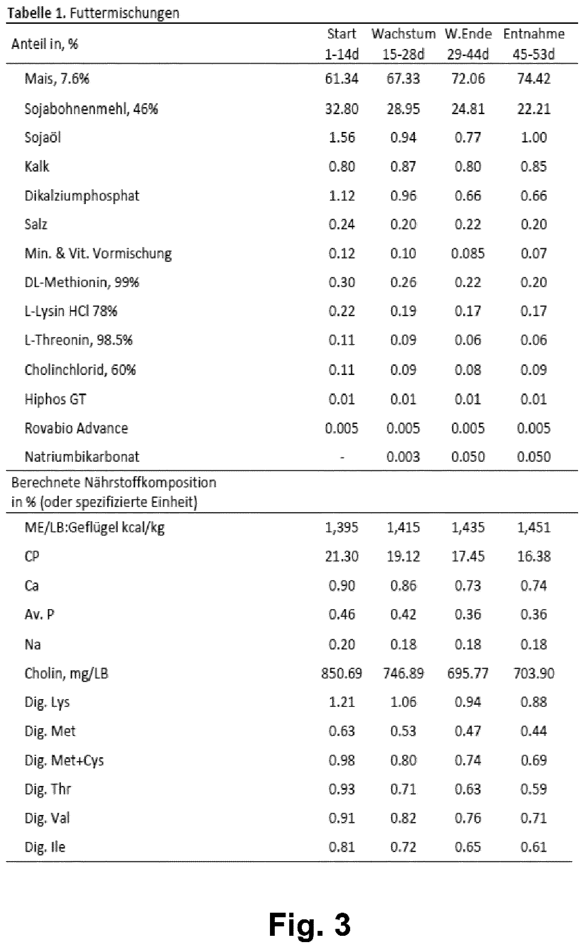

Es wurde ein altes Einstreumaterial verwendet, um darüber hinaus eine natürlichere Infektion mit in der Umwelt natürlicherweise vorkommenden Kokzidien und Bakterien zu bewirken. Der Boden der Versuchsabteile bzw. des Versuchsgebäudes war mit Erde/Schmutzfußboden versehen. Als Einstreu wurden Kiefernhobelspäne verwendet, auf welchen bereits drei Generationen Masthähnchen gehalten wurden. Jeder der Tiergruppen wurde Wasser und Futter ad libitum (zur freien Verfügung) bereitgestellt. Die Masthähnchen wurden in Abhängigkeit von ihrem Alter mit vier unterschiedlichen Varianten eines Mais-Soja-Futters gefüttert, die ausnahmslos pflanzlich waren und deren Zusammensetzung aus der Tabelle 1 in Figur 3 hervorgeht.

Während der gesamten Studie wurden dem Tierfutter der Tiergruppen, die eine Kombinationstherapie aus Isochinolinalkaloiden und einem Kokzidiostatikum oder einem Kokzidioseimpfstoff erhielten, die entsprechende Substanzkombination hinzugefügt. Das Wasser wurde nicht behandelt. Die dem Futter der Tiergruppen zugesetzten Isochinolinalkaloide bestanden aus einer Isochinolinalkaloidmischung, die zu ca. 50% aus Sanguinarin und zu ca. 50% aus Chelerythrin bestand.

Die Tiergruppen wurden wie folgt behandelt:
1. Kontrollgruppe (nicht aktiv infiziert, kein Futtermittelzusatz oder Impfstoff)
2. infizierte Kontrollgruppe (mit Kokzidien infiziert, kein Futtermittelzusatz oder Impfstoff)
3. infiziert wie Gruppe 2, behandelt mit nicht-ionophorem Antibiotikum (Nicarbazin - Nicarmix 100 ppm, Phibro)
4. infiziert wie Gruppe 2, behandelt mit ionophorem Antibiotikum (Salinomycin (Sacox 66 ppm, Huvepharma)
5. infiziert wie Gruppe 2, behandelt mit Impfstoff (Fortegra, MSD, wie Coccivac B52)
6. infiziert wie Gruppe 2, behandelt mit nicht-ionophorem Antibiotikum + 1 mg Isochinolinalkaloidmischung/kg Futter
7. infiziert wie Gruppe 2, behandelt mit nicht-ionophorem Antibiotikum + 2 mg Isochinolinalkaloidmischung/kg Futter
8. infiziert wie Gruppe 2, behandelt mit ionophorem Antibiotikum + 1 mg Isochinolinalkaloidmischung/kg Futter
9. infiziert wie Gruppe 2, behandelt mit ionophorem Antibiotikum + 2 mg Isochinolinalkaloidmischung/kg Futter
10. infiziert wie Gruppe 2, behandelt mit Impfstoff + 1 mg Isochinolinalkaloidmischung/kg Futter
11. infiziert wie Gruppe 2, behandelt mit Impfstoff + 2 mg Isochinolinalkaloidmischung/kg Futter

Die Isochinolinalkaloide (50% Sanguinarin, 50% Chelerythrin) basieren auf einer ethanolischen Extraktion einer Mohnpflanze. Die Basisquelle der Alkaloide sind botanischer Natur und stammen entweder aus der Mohngattung Sanguinaria canadensis oder Macleaya Cordata. Das geerntete Material wird durch einen Säureaufschluss des die Alkaloide enthaltenden Fasermaterials vorbehandelt, indem eine flüssige pflanzenzellwand-aufbrechende Säure, vorzugsweise Salzsäure und Cellulase-Enzyme, zugegeben wird, um die faserige Zellstruktur, die die Alkaloide enthält, aufzubrechen. Nach dem säure-enzymatischen Aufschluss des Pflanzenmaterials wird die Säure mit einem Neutralisationsmittel, vorzugsweise Natriumhydroxid oder einer Calciumhydroxid-Brühe, neutralisiert. Die Alkaloide werden dann aus dem pflanzlichen Fasermaterial gelöst und können aus der aufbereiteten pflanzlichen Biomasse durch Zugabe eines Lösungsmittels, vorzugsweise Ethanol, extrahiert werden. Der Alkaloid-Ethanol-Extrakt ist eine rot gefärbte, flüssige Lösung. In einem weiteren Schritt folgt eine Kristallisation durch Zugabe von Salzsäure oder Schwefelsäure. Das Ethanol wird durch einen Trocknungsprozess in einem Ofen oder einer anderen Sprühtrocknungstechnologie entfernt und das Endprodukt wird durch Mikronisierung zum Produkt hergestellt.

Das nicht-ionophore Antibiotikum der Gruppe 6 und 7 ist identisch zu dem der Gruppe 3. Das ionophore Antibiotikum der Gruppe 8 und 9 ist identisch zu dem der Gruppe 4. Die Isochinolinalkaloide wurden den Tiergruppen 6-11, das nicht-ionophore Antibiotikum den Tiergruppen 3, 6 und 7, und das ionophore Antibiotikum den Tiergruppen 4, 8 und 9 jeweils über das Futter verabreicht. Die Impfung bei den Tiergruppen 5, 10 und 11 erfolgte über das Auge, wie in Figur 2 dargestellt. Das Impfmittel war also nicht dem Futter beigemischt, dies kann aber eine alternativ mögliche Verabreichungsform darstellen.

Alle Daten wurden mittels einer Einweg-ANOVA mit SAS nach Prüfung auf Homoskedastizität analysiert. Daten, die keine Normalverteilung aufwiesen, wurden transformiert (log10). Ausreißer (außerhalb des +-2,5 SD-Bereichs) wurden aus der Datenbank entfernt. Die Ergebnisse der Kokzidiose-Score-Läsionen wurden mit der Statistiksoftware Statistix 9 ausgewertet und mit dem Shapiro-Wilk-Normalitätstest analysiert. Die parametrischen Daten wurden mit der Varianzanalyse (ANOVA) und dem Tukey-Test für die Mittelwerte mit einer signifikanten Differenz (P<0,05) unterlegt. Nichtparametrische Daten wurden dem Kruskal-Wallis-Test unterzogen (P<0,05).

Als Nullhypothese gilt die Annahme, dass kein Zusammenhang zwischen dem gemessenen Parameter und der Behandlungsvariante relativ zur Behandlung einer Vergleichsgruppe besteht und eventuelle Abweicher folglich "zufällig" sind. Der P-Wert (nach R. A. Fisher) gibt dabei jeweils die Wahrscheinlichkeit dafür an, dass - unter der Bedingung, dass die Nullhypothese in Wirklichkeit gilt - der beobachtete Wert der Prüfgröße erhalten wird. Der p-Wert kann als das kleinste Signifikanzniveau interpretiert werden, bei dem die Nullhypothese gerade noch verworfen werden kann. Ist der p-Wert "klein" (kleiner als ein vorgegebenes Signifikanzniveau; allgemein < 0,05), so lässt sich die Nullhypothese ablehnen. In diesem Fall kann man davon ausgehen, dass die Alternativhypothese gilt und damit ein bestimmter Zusammenhang besteht (z. B. eine bestimmte Behandlung wirksam ist). Wenn die Nullhypothese zugunsten der Alternativhypothese verworfen wird, wird das Resultat als "statistisch signifikant" bezeichnet.

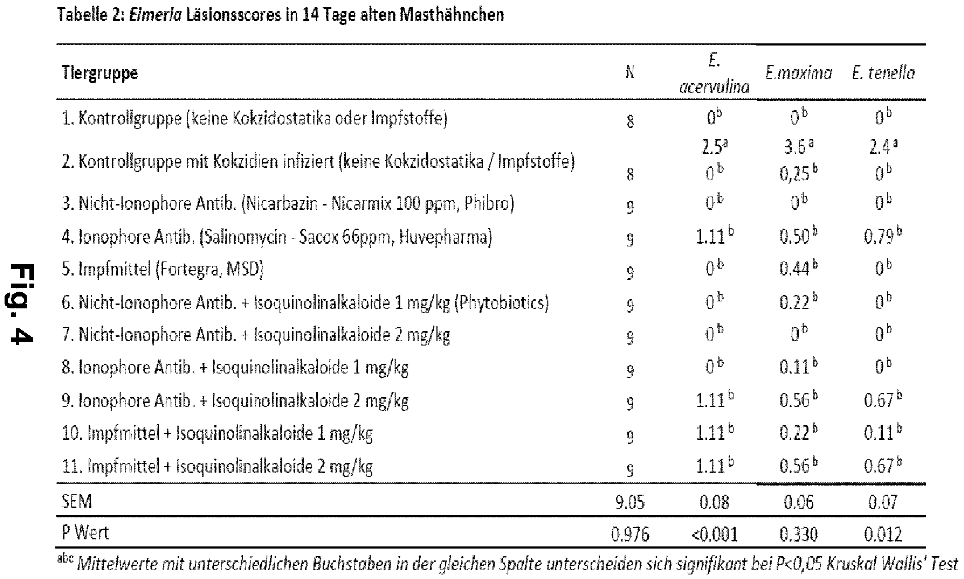

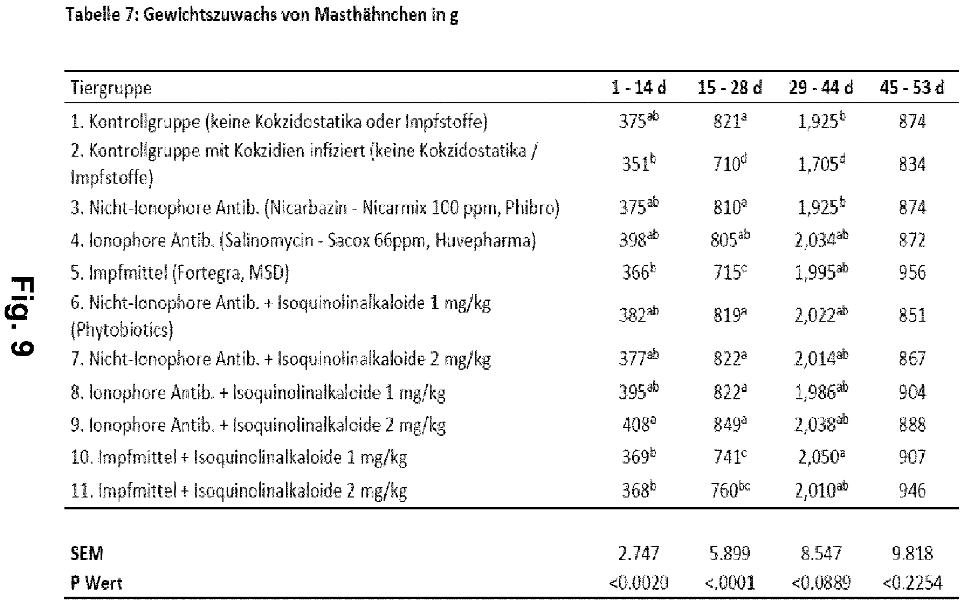

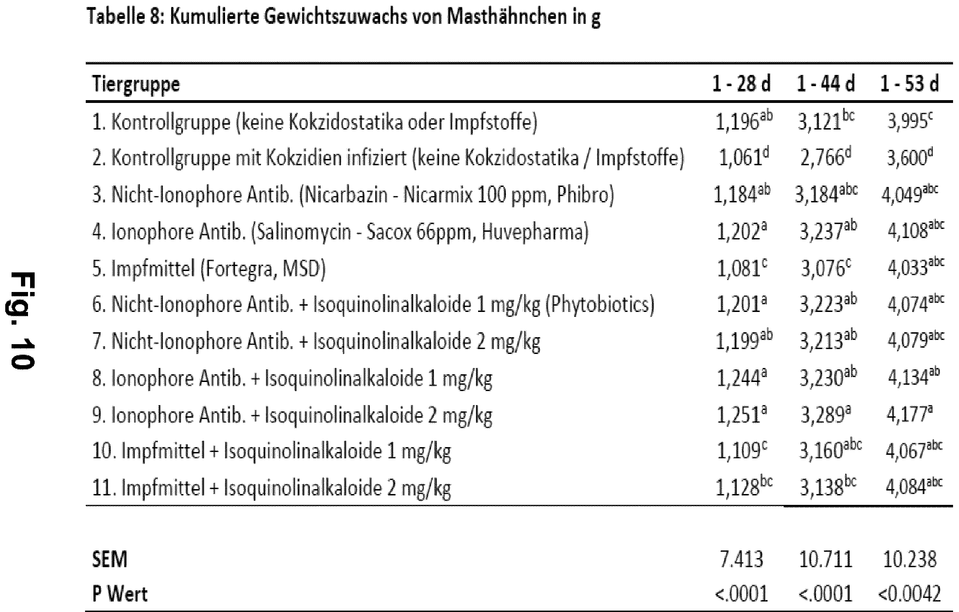

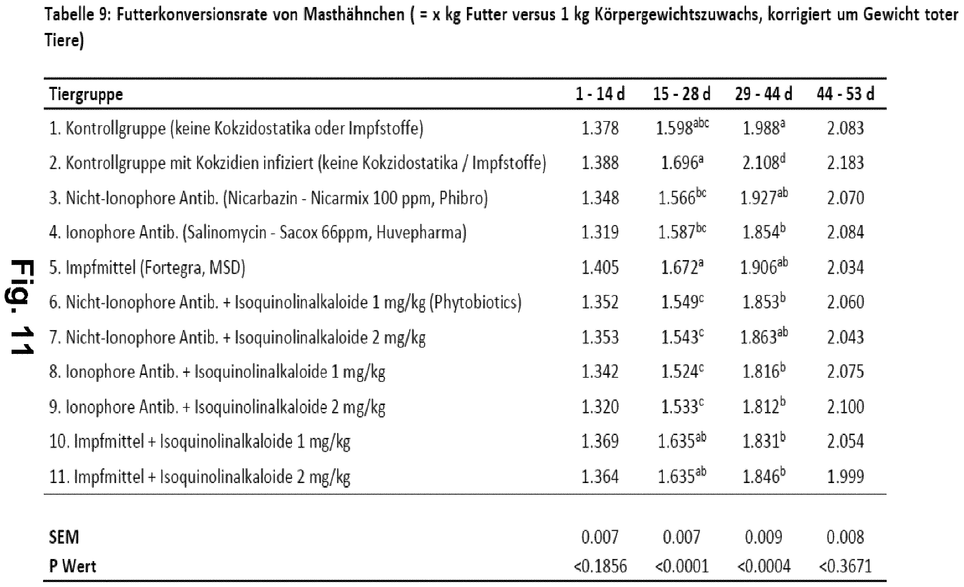

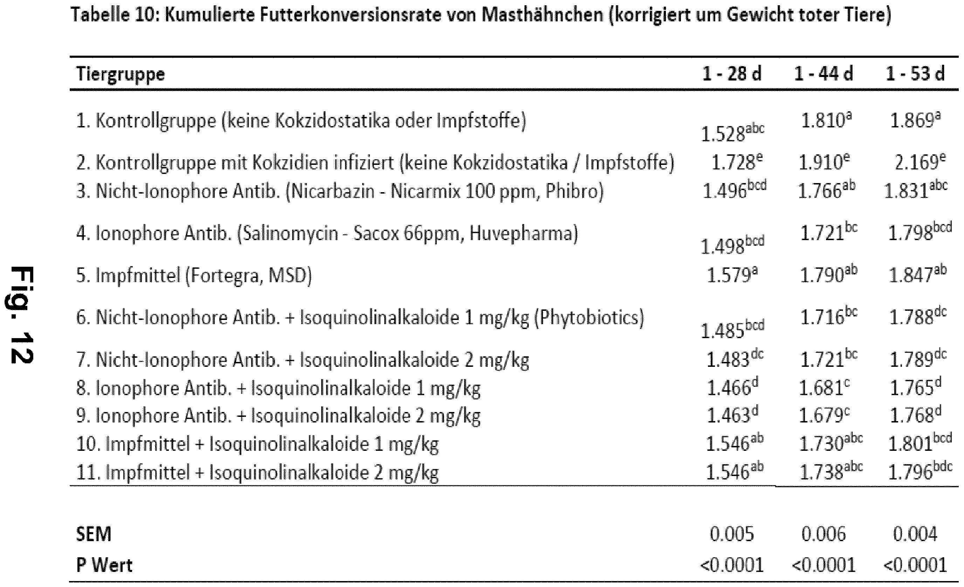

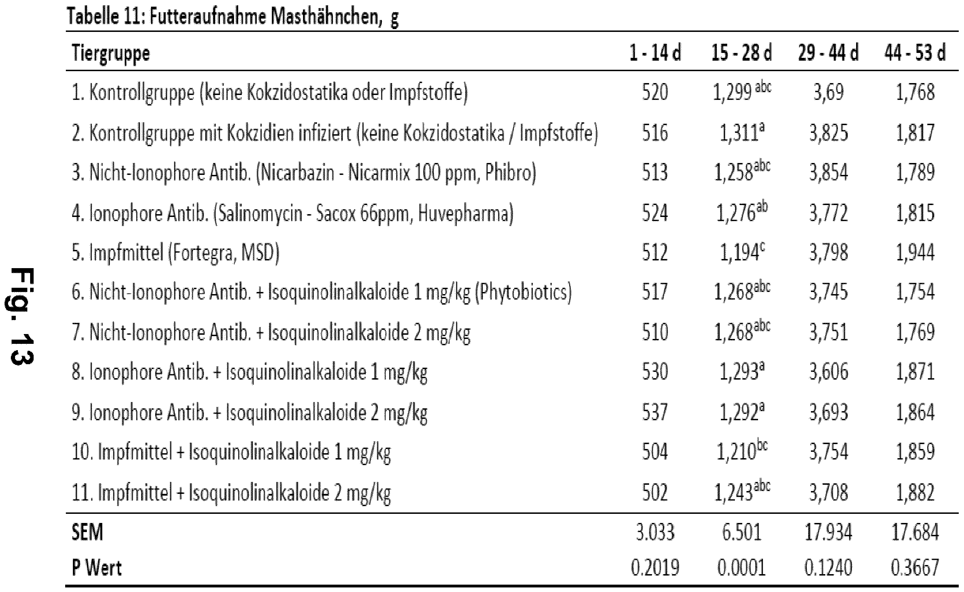

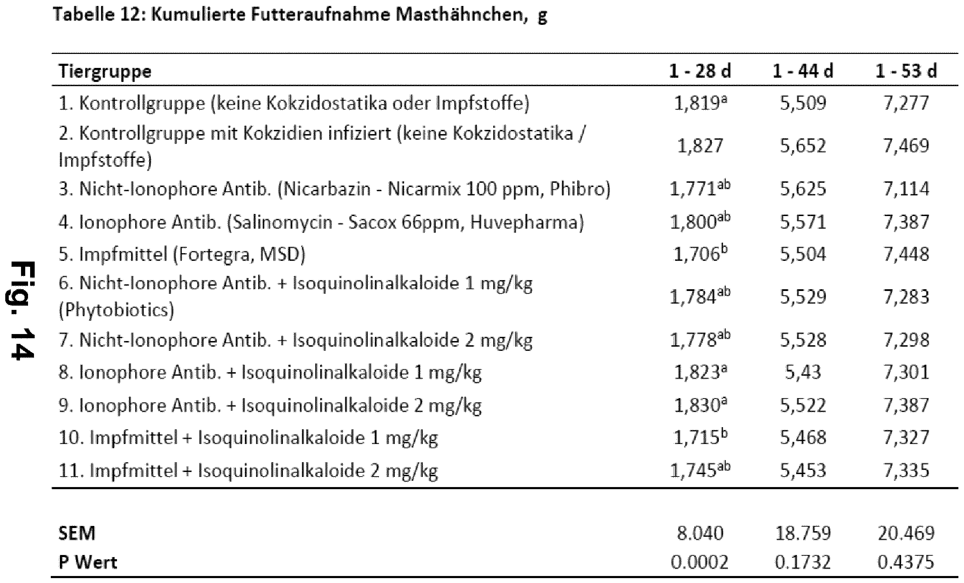

Die nachfolgenden Versuchsdaten zeigen, dass eine Kombinationstherapie aus Isochinolinalkaloiden und Kokzidostatika bzw. Kokzidioseimpfstoffen eine stärkere antikokzidale Wirkung hat als eine Monotherapie mit den entsprechenden Kokzidostatika bzw. Impfmitteln. Ersichtlich ist dies insbesondere anhand von kokzidien-induzierten Läsionsscores, Eimeria (Coccidia) Oozystenzählung und die Mortalität der betroffenen Tiere (Figuren 4-8). Die nachfolgenden Messwerte stellen die wichtigsten Parameter der Kokzidioseprävention bzw. -behandlung dar. Ein gutes Präventionsprogramm führt auch zu einer besseren Lebensleistung der Vögel als Folge der geringeren Infektionsrate der verschiedenen Darmkompartimente mit den Eimeria-Arten. Zu diesen indirekten positiven Effekten der besseren Darmgesundheit gehört auch eine bessere Futterverwertungsrate, was für den Landwirt von größtem kommerziellen Interesse ist (Figuren 9-14).

Figur 3 zeigt eine Tabelle mit Komponenten der vier verwendeten Futtermischungen während der Testlaufzeit. Das Futter "Start" wurde an den Tagen 1-14 verabreicht, das Futter "Wachstum" an den Tagen 15-28, das Futter "Wachstumsende" an den Tagen 29-44, und das Futter "Entnahme" an den Tagen 45-53.

Figuren 4, 5 und 6 zeigen jeweils eine Tabelle mit Eimeria Läsionsscores der getesteten Masthähnchengruppen im Alter von 14, 28 und 42 Tagen.

Eimerien sind eine Gattung von apikomplexen Parasiten, die verschiedene Arten umfasst, die die Krankheit Kokzidiose bei Tieren wie Rindern, Geflügel und kleineren Wiederkäuern, einschließlich Schafen und Ziegen, verursachen können. Die Arten dieser Gattung infizieren eine Vielzahl von Wirten, darunter Geflügel, Rinder, Schafe, Ziegen, aber auch Fische, Fledermäuse, Schildkröten, Robben, Nagetiere, Lamas und Alpakas. Die häufigsten Eimeria-Arten, die Kokzidiose bei Rindern verursachen, sind E. bovis, E. zuernii und E. auburnensis. Bei einem jungen, anfälligen Kalb können schätzungsweise nur 50.000 infektiöse Oozysten eine schwere Krankheit verursachen. Eimerieninfektionen sind besonders schädlich für die Geflügelindustrie und kosten die Vereinigten Staaten jährlich mehr als 1,5 Milliarden Dollar an Verlusten. Die wirtschaftlich wichtigsten Arten bei Geflügel sind E. tenella, E. acervulina und E. maxima.

Die Darmgesundheit wurde durch makroskopische Untersuchung von 1 zufällig sezierten Vogel pro Versuchsabteil untersucht. Die Darmtrakte wurden individuell untersucht und auf das Auftreten von Läsionen, die auf Kokzidiose und / oder nekrotisierende Enteritis hindeuten, untersucht und bewertet.

Die Tabelle der Figur 4 zeigt, dass die Infektion der zweiten Kontrollgruppe ("Gruppe 2") mit Kokzidien erfolgreich durchgeführt wurde: die jeweiligen Eimeria-Läsionsscores in dieser Gruppe 2 waren im Vergleich zu den Gruppen mit antikozidialer Intervention signifikant höher. Die Gruppen mit der kombinierten Anwendung des Antikozidienmedikaments oder Impfstoffs und der Isochinolinalkaloide hatten ähnliche oder zahlenmäßig bessere Läsionsscores als die Gruppen, in denen das Medikament als Monotherapie, also ohne die Isochinolinalkaloide, angewendet wurde.

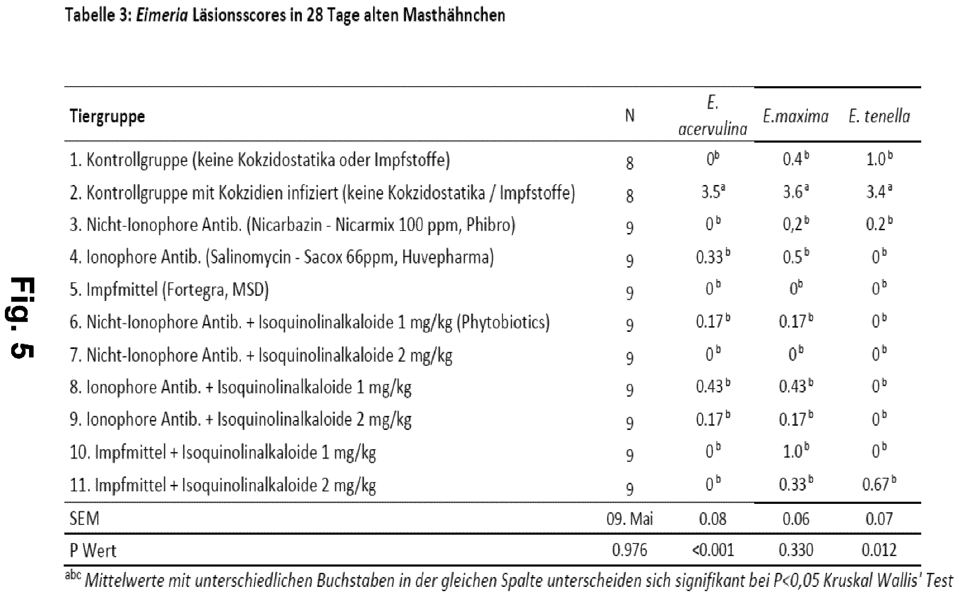

Die Tabelle der Figur 5 zeigt die Läsionsscores am 28. Lebenstag der Masthähnchen. In diesem Alter wirt allgemein die höchste Kokzidien-Infektionsrate und Ausprägung beobachtet. Die Tabelle zeigt, dass die Infektion mit Kokzidien erfolgreich durchgeführt wurde (Gruppe 2) und die jeweiligen Eimeria-Läsionen-Scores in dieser Gruppe signifikant höher waren als in den Gruppen mit antikozidialer Intervention. Die Gruppen mit der kombinierten Anwendung des Antikozidienmedikaments oder Impfstoffs und der Isochinolin-Alkaloide hatten ähnliche oder zahlenmäßig bessere Läsionsscores als die Gruppen, in denen das Medikament ohne die Isochinolinalkaloide angewendet wurde.

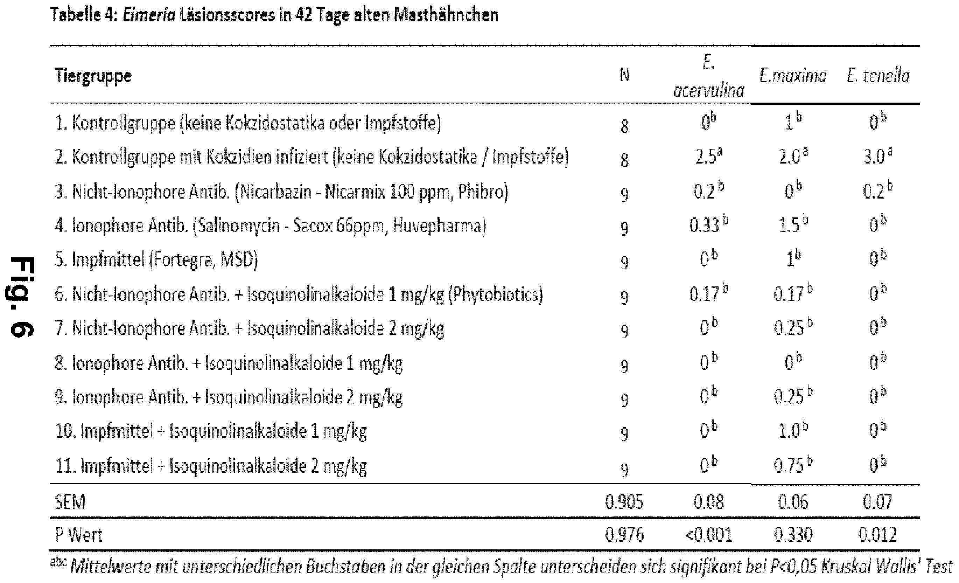

Die Tabelle der Figur 6 zeigt die Läsiionsscores am 42. Lebenstag der Masthähnchen. Ein Zeitraum von 42 Tagen entspricht der "Lebenszeit" einer typischen Kokzidiose-Infektion. Auch für diesen Zeitpunkt wurde beobachtet, dass Tiergruppen mit der kombinierten Anwendung des Antikozidienmedikaments oder Impfstoffs und der Isochinolin-Alkaloide ähnliche oder zahlenmäßig bessere Läsionsscores hatten als die Gruppen, in denen das Medikament ohne die Isochinolinalkaloide angewendet wurde.

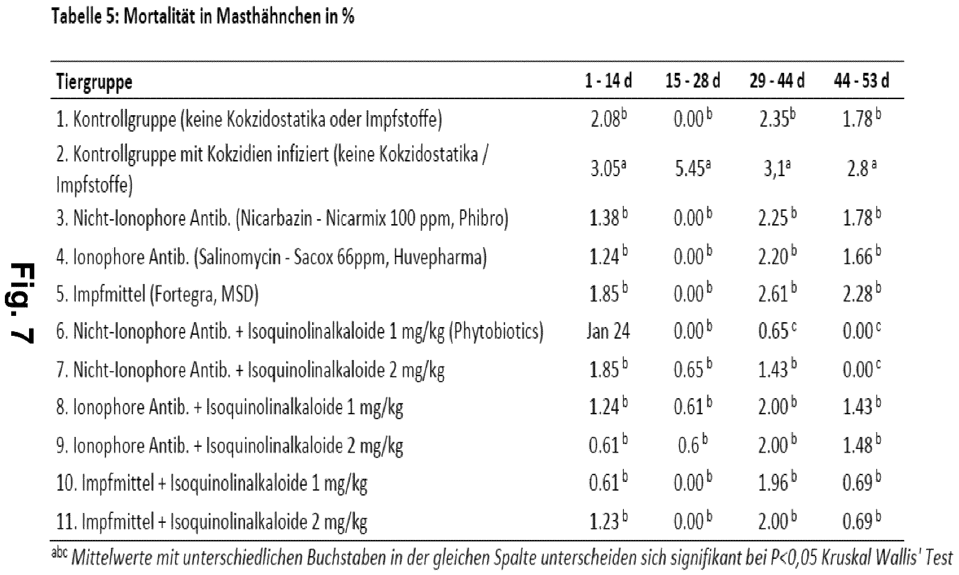

Die Tabelle der Figur 7 zeigt die Mortalität der Masthähnchen dieses Tests. Die Mortalität wurde während des gesamten Versuchszeitraums täglich aufgezeichnet. Die Mortatlität ist sowohl ein wichtiger medizinischer als auch ein ökonomischer Parameter einer Kokzidiose-Infektion. Die Sterblichkeitsrate der Gruppe 2 beweist, dass die Infektion der Gruppen 2-11 mit Kokzidien erfolgreich durchgeführt wurde. Zudem waren auch die Läsionsscores in Gruppe 2 signivikant höher als in anderen Grupppen.

Die Tiergruppen mit einer "Kombitherapie" (Isochinolinalkaloid + Kokzidiostatikum oder Impfstoff) hatten in der wirtschaftlich kritischsten Phase von Tag 29 bis 44 eine ähnliche oder signifikant niedrigere Mortalität als die Gruppen, in denen das Medikament ohne die Isochinolin-Alkaloide angewendet wurde. Ein enormer ökonomischer Nutzen wurde durch die Kombination des chemischen Medikaments Nicarbazin mit den Isoquinolin-Alkaloiden in der Mortalität während der letzten Tage nachgewiesen, als die Vögel bereits ihr gesamtes Futter verbraucht und somit bereits eine hohe Investition getätigt hatten. Die Kombination reduzierte die Mortalität auf 0%, während alle anderen Gruppen eine leicht niedrige oder moderate Mortalität auch unter einem Kokzidiosepräventionsprogramm hatten. Das Nicarbazin allein war signifikant weniger wirksam als die Kombination von Nicarbazin mit den Isochinolinalkaloiden.

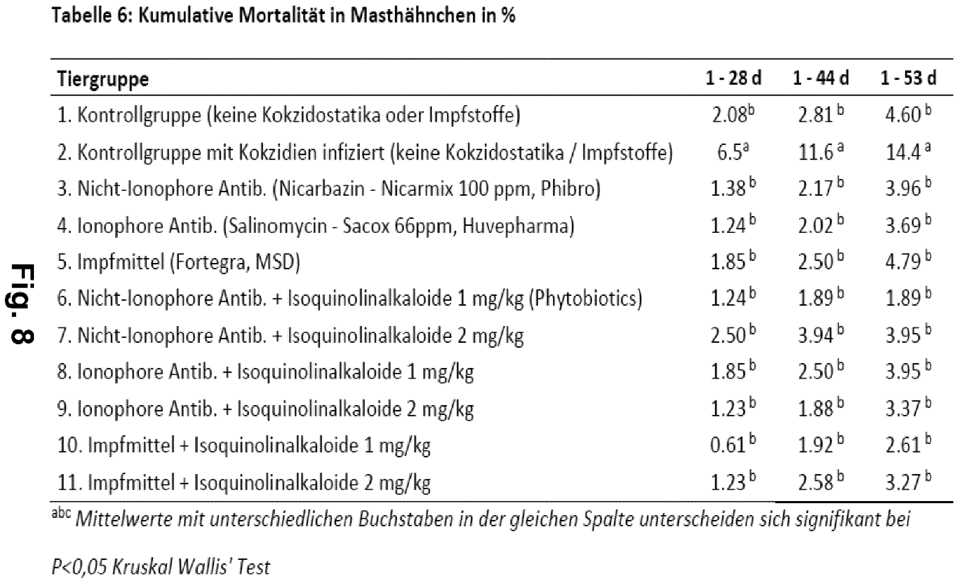

Die Tabelle der Figur 8 zeigt die kumulative Mortalität der Masthähnchen des Versuchs. Figur 8 lässt erkennen, dass die Infektion mit Kokzidien erfolgreich durchgeführt wurde (Gruppe 2) und die entsprechenden Eimeria-Läsionen in dieser Gruppe signifikant höher waren als in den Gruppen mit antikozidialer Intervention. Die Gruppen mit der kombinierten Anwendung des Antikozidienmedikaments oder Impfstoffs und der Isochinolinalkaloide hatten eine ähnliche oder signifikant niedrigere Mortalität als die Gruppen, in denen das Medikament ohne die Isochinolin-Alkaloide angewendet wurde. Die deutlich niedrigste Mortalität aller Gruppen ergab sich bei der Kombination von Nicarbazin mit den Isochinolinalkaloiden.

Die Ergebnisse der Läsionsscore-Analyse des Darmgewebes der Vögel in den verschiedenen Mastphasen beweisen eindeutig, dass die Infektion stattgefunden hat und erfolgreich in die Vögel eingebracht wurde, die Differenz der Läsionsscore zwischen Gruppe 2 und den anderen zeigt diesen Effekt.

Alle Interventionen zeigen eine verbesserte Darmgesundheit, ersichtlich durch geringere Läsionsscore-Zahlen und sind somit ein Beweis für das Konzept.

Weitere molekularbiologische Untersuchungen bezüglich der Blutplasmakonzentrationen von FITC-Dextran-, TNF-alpha und IL-10 (hier nicht gezeigt) haben die erhöhte Wirksamkeit eines Kombipräparates mit Isochinolinalkaloiden bestätigt.

FITC-Dextran- und TNF-alpha Konzentrationen der getesteten Masthähnchengruppen sind Biomarker, die Auskunft über die Stärke des Kokzidienbefalls und der hierdurch induzierten körperlichen Reaktionen geben.

FITC-Dextran ist ein Marker für die Permeabilität des Darms, die bei Kokzidiose erhöht sein kann. Einmal oral verabreicht, durchquert dieser fluoreszierende Marker den Magen-DarmTrakt und durchquert passiv das Darmepithel. Die mit einem Fluorimeter leicht zu bestimmende Konzentration von 4-kDa FITC-Dextran im Blutplasma stellt ein Maß für die parazelluläre Permeabilität des Darmepithels dar.

Tumornekrosefaktor (kurz: TNF) ist ein multifunktionaler Signalstoff (Zytokin) des Immunsystems, welcher bei lokalen und systemischen Entzündungen beteiligt ist. Er ist ein Krankheitsindikator bezüglich Kokzidiose.

Interleukin-10 (oder kurz: IL10) gehört innerhalb der Zytokine zu den Interleukinen. Es hat zahlreiche Funktionen in der Regulation des Immunsystems. Es ist eines der wichtigsten antientzündlichen Zytokine und wichtig zur Entwicklung der Immuntoleranz. Interleukin-10 ist ein wesentlicher Immunmodulator im Intestinaltrakt. Ein Mangel geht mit einer Veranlagung zu chronisch entzündlichen Darmerkrankungen wie Morbus Crohn oder Colitis ulcerosa einher. Ein erhöhter IL-10 Wert ist ein Krankheitsindikator bezüglich Kokzidiose.

Zur Messung der FITC-Dextran Werte erhielten die Masthähnchen oral am Tag 28 (8. März) eine Dosis von 4,16 mg FITC-d (MW 3.000-5.000, FD4, Sigma Aldrich Ca., Saint Louis, MO) pro Vogel. Es wurde Blut 2,5 Stunden danach entnommen. Die Blutproben wurden für 3 Stunden bei Raumtemperatur aufbewahrt, um die Gerinnung zu ermöglichen, und dann zur Separation des Seerums zentrifugiert (500 x g für 15 min). Das Serum wurde verdünnt (1: 1 PBS) und bei einer Wellenlänge von 528 nm gemessen. Die Konzentration von FITC-d pro mL Serum wurde dann auf der Basis eines Standards berechnet (Baxter et al., 2017).

Ebenfalls am Tag 28 (8. März) wurden vier weitere Masthähnchen nach dem Zufallsprinzip aus jeder Tiergruppe ausgewählt und Blutproben aus den Flügelvenen in heparinisierten Röhren entnommen. Die Blutproben wurden auf Eis gelagert, bei 2.500 x g für 10 min bei 4 °C zentrifugiert und das Plasma bis dahin bei -80 °C gelagert. Es wurden die Plasmakonzentrationen für TNF-alpha und IL-10 alpha gemessen. Es stellte sich heraus, dass eine Kombitherapie bzw. Kombiimpfung unter Verwendung von Isochinolinalkaloiden die Plasmawerte sowohl von TNF-alpha also auch von IL-10 gegenüber der Monotherapie bzw. alleinigen Impung senkt, teilweise sogar deutlich senkt. Dies lässt auf eine verbesserte Wirksamkeit einer Kombinationsimpfung mit Isochinolinalkaloid schließen im Verlgeich zur reinen Impfung ohne Isochinolinalkaloide.

Die Ergebnisse der in Figuren 1-14 veranschaulichten Studie zeigen, dass ein Kokzidiosepräventionskonzept, bei dem ein Impfstoff oder ein Kokzidiostatikum in Kombination mit Isochinolinalkaloiden verabreicht wird, einen verbesserten Schutz vor bzw. eine verbesserte Bekämpfung einer bestehenden Kokzidiose bewirkt im Vergleich zu einer Verabreichung als Monotherapie ohne Isochinolinalkaloide. Darüberhinaus konnte z.B. für das nicht-ionophore Antibiotikum Nicarbazin gezeigt werden, dass zumindest in Kombination mit Isochinolinalkaloiden auch ein nicht-ionophores Antibiotikum ein wirksames Instrument für großflächige Geflügelmastbetriebe ist, um diese wirtschaftlich bedeutendste Krankheit zu verhindern.

Die derzeit in 85 % aller Betriebe weltweit angewandte Praxis ist der Einsatz von ionophoren Antibiotika als Kokzidiostatikum (Monensin, Narasin, Maduramycin, Salinomycin, Lasalocid etc.) oder Kombinationen von antimikrobiellen Medikamenten mit nicht-ionophoren Antibiotika wie die Mischung Maxiban (Narasin & Nicarbazin). Der Einsatz von Antibiotika, insbesondere von ionophoren Antibiotka, hat aber den großen Nachteil, dass sie die Verbreitung von Resistenzmutationen fördern. Aufgrund des Drucks der Verbraucher, die Verwendung von resistenzinduzierenden antimikrobiellen Mitteln in landwirtschaftlichen Betrieben zu reduzieren, liegt es im Interesse der Landwirte, neue Interventionssysteme gegen Kokzidiose zu entwickeln, die ebenso gut wie die bekannten ionophoren Antibiotika wirken, ohne die besagten Nachteile zu haben.

Ausführungsformen der Erfindung erlauben es, Kokzidiose mit Substanzen zu bekämpfen (Impfstoffe, nicht-ionophore Antibiotika), die nicht wie die heute als Kokzidiostatika verwendeten ionophoren Antibiotika Resistenzen induzieren. Ausführungsformen der Erfindung können einen wirksamen Kokzidioseschutz bewirken, ohne die Wirksamkeit der Behandlung zu reduzieren, teilweise wird die Wirksamkeit gegenüber der Monotherapie sogar deutlich erhöht.

Die Ausführungen zeigen dass a) eine Kombination von Nicarbazin in kommerziell genutzter Dosierung mit Isoquinolinalkaloiden sowie b) eine Kombination des Impfstoffs zusammen mit den Isoquinolinalkaloiden die gleiche Kokzidiose-verhindernde Wirkung wie das antimikrobielle Antikozidienmittel Salinomycin hat, jedoch ohne die negativen Folgen einer Resistenzinduktion.

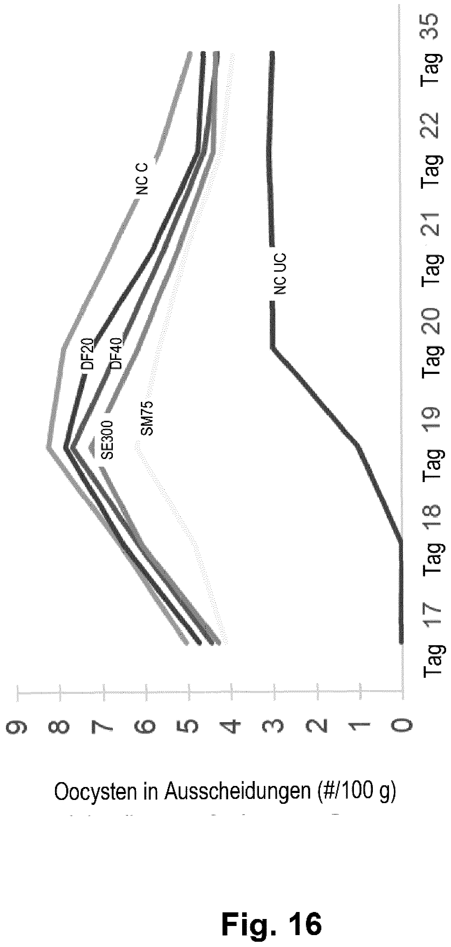

Figur 16 zeigt Läsionsscores verschiedener Masthähnchengruppen in einer weiteren Studie. Es wurden sechs Gruppen von Masthänchen gebildet, die unterschiedliche Futterzusätze bekamen und abgesehen von einer Kontrollgruppe I mit Kokzidiose infiziert wurden. Die Studiendauer betrug 42 Tage ausgehend vom Tag des Schlüpfens. Es wurden nur männliche Mastküken der Linie Ross 308 verwendet. Als Grundfutter wurde zu Beginn des Versuchs (Tag 1-21) Maisfutter, später ein Futter auf Basis von Weizen- und Sojaschrot verwendet. Jede der in der untenstehenden Tabelle gelisteten Tiergruppen bestand aus 80 Tieren, die zu je 20 Vögeln in vier Versuchsabteilen gehalten wurden.

Am 14. Tag des Versuchs wurden die Tiere aller Gruppen bis auf die Kontrollgruppe I (NC no C) oral mit sporulierten Oozysten von 35.000 E. acervulina, 10.000 E. maxima und 10.000 E. tenella infiziert.

| TIERGRUPPE | BEHANDLUNG | INFEKTION MIT KOKZIDIEN |
|---|---|---|
| NC no C | Kontrollgruppe I: Keine Futteradditive, keine Infektion mit Kokkzidien | Nein |
| ICC | Kontrollgruppe II: Keine Futteradditive, Infektion mit Kokzidien | + |
| ORC | Futteradditiv: Oregano (Oreganoöl Präparation, 100 mg/kg), Infektion mit Kokzidien | + |
| A-Cox C | Futteradditiv: Adicox (Mischung von antiparasitär wirksamen Kräutern 200 mg/kg), Infektion mit Kokzidien | + |
| Nic 100 C | Futteradditiv: Nicarbazin (100 mg/kg), Infektion mit Kokzidien | + |
| Iso-Nic C | Futteradditiv: Isochinolinalkaloide (50% Sanguinarin, 50% Chelerythrin) (1 mg/kg)+ Nicarbazin (100 mg/kg), Infektion mit Kokzidien | + |

Während einer Kokzidiose entwickelten die Masthähnchen in der Regel einen erhöhten Läsionsscore als Folge der Ausbreitung des Eimeria Erregers. Die Ergebnisse zeigen im Vergleich zur infizierten IC-Gruppe signifikant verringerte Läsionsscores für die Gruppen "Iso-Nic" und "Nic 100". Die Kombination Iso-Nic erwies sich als die beste Lösung in einem Kokzidiosepräventionsprogramm, gemessen als Läsionsscoring-Zahlen gegenüber allen anderen potenziellen chemischen oder natürlichen Lösungen wie Nicarbazin, Oregano oder Adicox.

**Figur 16** zeigt die beobachtete Oozystenzahl in Ausscheidungen der Tiere der weiteren Studie. Kokzidien vermehren sich ungeschlechtlich und geschlechtlich. Im Anschluss an die ungeschlechtliche Vermehrungsphase (Schizogonie) bilden sich Geschlechtszellen (Gametogonie), nämlich große plasmareiche Makrogameten und kleine begeißelte Mikrogameten, und es vollzieht sich eine geschlechtliche Vermehrung. Die befruchtete weibliche Zelle (Zygote) umgibt sich mit einer Hülle (Enzystierung) und wird zur Oozyste. Sie wird mit dem Kot des Wirtes ausgeschieden und ist ein verlässlicher Indikator der Stärke einer akuten Kokzidiose.

Während des Spitzenwertes der Oozystenaussceidung (Tag 18 bis Tag 21) hatte die Iso-Nic Tiergruppe eine 10 bis 100 mal niedrigere Oozystenzahl pro gr Kot als die Gruppen, die mit anderen antikozidialen Interventionen behandelt wurden.

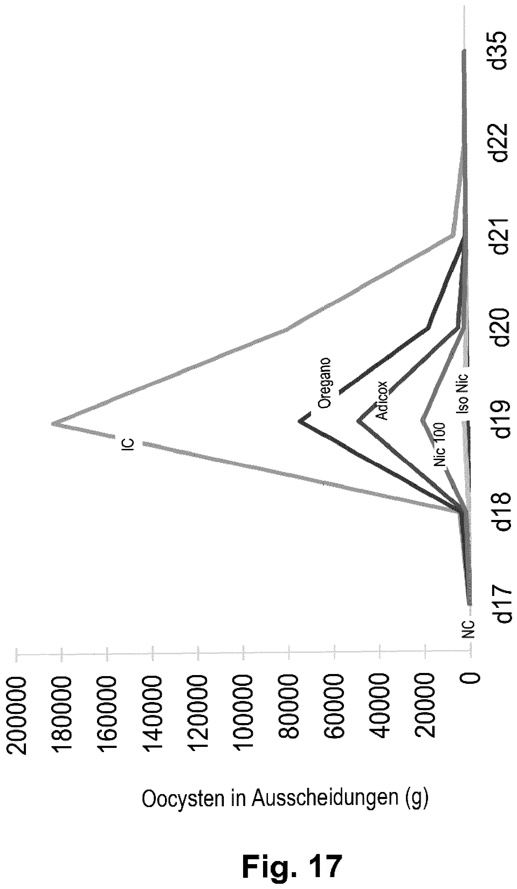

**Figur 17** zeigt die beobachtete Oozystenmasse in Ausscheidungen der Tiere der weiteren Studie. Während des Spitzenwertes der Oozystenausscheidung (Tag 18 bis Tag 19) hatte die Iso-Nic Kombinationsgruppe eine Oozystenzahl pro gr Kot, die 10 mal niedriger war als die von Nic allein und 100 mal niedriger als die der infizierten, nicht behandelten Gruppe IC.

## Patentansprüche

1. Substanzkombination zur Behandlung von Kokzidiose bei Nutztieren oder Hobbytieren, wobei die Substanzkombination Isochinolinalkaloide enthält und wobei die Substanzkombination zudem ein Kokzidiostatikum oder ein Kokzidioseimpfmittel enthält.

2. Die Substanzkombination nach Anspruch 1, wobei die Substanzkombination ausgebildet ist als Medikament, Tiernahrung, Tränkwasser oder Tiernahrungsergänzungsmittel.

3. Die Substanzkombination nach einem der vorhergehenden Ansprüche, wobei die Behandlung eine vorbeugende Behandlung oder eine Akutbehandlung ist.

4. Die Substanzkombination nach einem der vorhergehenden Ansprüche, wobei die Behandlung insbesondere der Behandlung von kokzidieninduzierten Darmentzündungen und/oder kokzidieninduzierten Durchfällen bei Nutztieren oder Hobbytieren dient.

5. Die Substanzkombination nach einem der vorhergehenden Ansprüche, wobei es sich bei dem Kokzidiostatikum um ein ionophores Antibiotikum handelt.

6. Die Substanzkombination nach Anspruch 5, wobei das ionophore Antibiotikum Salinomycin, Monensin, oder Narasin ist.

7. Die Substanzkombination nach einem der vorhergehenden Ansprüche 1-4, wobei es sich bei dem Kokzidiostatikum um ein nicht-ionophores Antibiotikum handelt.

8. Die Substanzkombination nach Anspruch 7, wobei das nicht-ionophore Antibiotikum Nicarbazin ist.

9. Die Substanzkombination nach Anspruch 8,
- wobei die Substanzkombination eine Tiernahrung oder ein Tränkwasser ist, wobei die Substanzkombination das Nicarbazin in einer Konzentration von 20 mg bis 500 mg, vorzugsweise von 50 mg bis 200 mg pro kg Tiernahrung enthält; oder
- wobei die Substanzkombination ein Tiernahrungsergänzungsmittel oder Medikament ist, welches das Nicarbazin in einer Konzentration enthält, die bei bestimmungsmäßiger Beimischung des Tiernahrungsergänzungsmittels oder des Medikaments zu einer Tiernahrung eine Tiernahrung mit einer Nicarbazinkonzentration von 20 mg bis 500 mg, vorzugsweise von 50 mg bis 200 mg pro kg Tiernahrung ergibt; oder
- wobei die Substanzkombination ein Tiernahrungsergänzungsmittel oder Medikament ist, welches das Nicarbazin in einer Konzentration enthält, die bei bestimmungsmäßiger Beimischung des Tiernahrungsergänzungsmittels oder des Medikaments zu einem Tränkwasser ein Tränkwasser mit einer Nicarbazinkonzentration von 20 mg bis 500 mg, vorzugsweise von 50 mg bis 200 mg pro Liter Tränkwasser ergibt.

10. Die Substanzkombination nach einem der vorhergehenden Ansprüche 1-4, wobei es sich bei dem Kokzidioseimpfmittel um einen Lebendimpfstoff handelt, der sporulierte Oozysten von einer oder mehreren Eimeria-Arten beinhaltet.

11. Die Substanzkombination nach einem der vorigen Ansprüche, welches Pflanzenmaterial der Gattung Sanguinaria, insbesondere Sanguinaria canadensis, und/oder der Gattung Argemone, insbesondere Argemone mexicana, und/oder der Gattung Chelidonium, insbesondere Chelidonium majus, und/oder der Gattung Macleaya, insbesondere Macleaya cordata oder eines Extraktes dieses Pflanzenmaterials enthält, wobei die Isochinolinalkaloide Bestandteil des Pflanzenmaterials sind.

12. Die Substanzkombination nach einem der vorigen Ansprüche, wobei die Isochinolinalkaloide Benzophenanthiridin-Alkaloide in physiologisch wirksamer Menge sind, insbesondere Sanguinarin, Chelerythrin oder eine Mischung dieser beiden.

13. Die Substanzkombination nach einem der vorigen Ansprüche,
- wobei die Substanzkombination eine Tiernahrung oder ein Tränkwasser ist, wobei die Substanzkombination die Isochinolinalkaloide in einer Konzentration von 0,1 mg bis 10 mg, vorzugsweise von 0,5 mg bis 5 mg pro kg Substanzkombination enthält; oder
- wobei die Substanzkombination ein Tiernahrungsergänzungsmittel oder Medikament ist, welches die Isochinolinalkaloide in einer Konzentration enthält, die bei bestimmungsmäßiger Beimischung der Substanzkombination in eine Tiernahrung eine Tiernahrung mit einer Isochinolinalkaloidkonzentration von 0,1 mg bis 10 mg, vorzugsweise von 0,5 mg bis 5 mg pro kg Tiernahrung ergibt; oder
- wobei die Substanzkombination ein Tiernahrungsergänzungsmittel oder Medikament ist, welches die Isochinolinalkaloide in einer Konzentration enthält, die bei bestimmungsmäßiger Beimischung der Substanzkombination in ein Tränkwasser ein Tränkwasser mit einer Isochinolinalkaloidkonzentration von 0,1 mg bis 10 mg, vorzugsweise von 0,5 mg bis 5 mg pro Liter Tränkwasser ergibt.

14. Die Substanzkombination nach einem der vorigen Ansprüche, wobei es sich bei den Nutztieren um Rinder, Kühe, Schweine, Pferde, Geflügel, Schafe, Ziegen, Kaninchen, oder Fische, und insbesondere um Geflügel handelt, und/oder wobei es sich bei den Hobbytieren um Hunde oder Katzen handelt.

15. Die Substanzkombination nach einem der vorhergehenden Ansprüche, wobei die Substanzkombination ausgebildet ist als Substanzmischung oder als ein Behandlungsset aus mindestens zwei Komponenten, wobei eine der Komponenten die Isochinolinalkaloide und die andere Komponente das Kokzidiostatikum oder das Kokzidioseimpfmittel enthält.

16. Verfahren zur Behandlung von Kokzidiose bei Nutztieren oder Hobbytieren, **dadurch gekennzeichnet, daß** den Nutztieren oder Hobbytieren eine Substanzkombination nach einem der Ansprüche 1-15 verfüttert wird.

17. Verfahren nach einem der vorigen Ansprüche,
- wobei die pro Tag verabreichte Menge an Isochinolinalkaloiden zwischen 0,02 und 3,0 mg pro kg Lebendgewicht der Nutztiere oder Hobbytiere, bevorzugt zwischen 0,02 und 1,0 mg pro kg Lebendgewicht der Nutztiere oder Hobbytiere liegt; und/oder
- wobei das Kokzidiostatikum Nicarbazin ist und wobei die pro Tag verabreichte Menge an Nicarbazin bei mindestens 2 mg Nicarbazin pro kg Lebendgewicht der Nutztiere oder Hobbytiere, bevorzugt zwischen 2 und 50 mg Nicarbazin pro kg Lebendgewicht der Nutztiere oder Hobbytiere liegt.

18. Verwendung einer Substanzkombination nach einem der Ansprüche 1 bis 15 zur vorbeugenden und/oder akuten Behandlung von Kokzidiose bei Nutztieren oder Hobbytieren.
